# EUROPEAN PATENT APPLICATION

(11) **EP 4 001 274 A1**
(43) Date of publication of application: **25.05.2022**
(21) Application number: 20840778.3
(22) Date of filing: 13.07.2020
(51) Int. Cl.: C07D 407/10, C07D 401/14, C07D 401/12, C07D 405/14, C07D 413/14, C07D 213/56, A61K 31/44, A61K 31/4545, A61K 31/444, A61P 35/00, A61P 37/02

(54) **BIPHENYL DERIVATIVES FOR BLOCKING PD-1/PD-L1 INTERACTION, PREPARATION METHOD THEREFOR AND USE THEREOF**

(30) Priority: 18.07.2019 CN 201910651570
(71) Applicant: Abbisko Therapeutics Co., Ltd., Shanghai 201203 (CN)
(72) Inventor: YANG, Fei, Shanghai 201203 (CN); ZHANG, Yongxian, Shanghai 201203 (CN); YU, Hongping, Shanghai 201203 (CN); CHEN, Zhui, Shanghai 201203 (CN); XU, Yaochang, Shanghai 201203 (CN)
(74) Representative: dompatent von Kreisler Selting Werner - Partnerschaft von Patent- und Rechtsanwälten mbB
(86) International application number: PCT/CN2020/101646
(87) International publication number: WO 2021/008491

(57) **Abstract**

Biphenyl derivatives having a structure of formula (I) and capable of blocking PD-1/PD-L1 interaction, a preparation method therefor and use thereof. The series of compounds can be widely used in the preparation of medicaments for preventing and/or treating cancers or tumors, immune-related disease and disorders, communicable diseases, infectious diseases or metabolic diseases mediated by PD-1/PD-L1 signal pathways, and are expected to be developed into a new generation of PD-1/PD-L1 inhibitors.

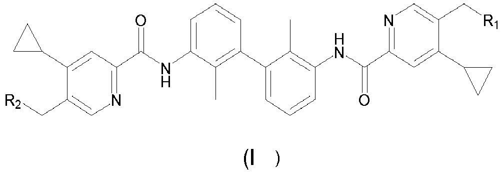

## Description

### TECHNICAL FIELD

The present invention belongs to the field of pharmaceutical synthesis, and particularly relates to biphenyl derivatives for blocking PD-1/ PD-L1 interaction, preparation method therefor and use thereof.

### BACKGROUND

The immune system plays a very important role in controlling and eliminating diseases, such as cancer. However, tumor cells are often able to develop a strategy for escaping or suppressing the monitoring of the immune system to promote their malignant growth. One very important mechanism is to change the expression of co-stimulatory and co-inhibitory immune checkpoint molecules on immunocytes. Blocking the signal pathway of immune checkpoint molecules, such as PD1, has been proved to be an extremely promising and effective therapy.

Programmed cell death protein 1 (PD-1), also known as CD279, is a receptor expressed on the surfaces of activated T cells, natural killer T cells, B cells and macrophages. The structure of PD-1 contains an extracellular domain similar to an immunoglobulin variable region, a transmembrane domain and an intracellular domain, wherein the intracellular domain contains two phosphorylation sites located in an immunoreceptor tyrosine kinase-based inhibitory domain and an immunoreceptor tyrosine kinase-based transduction domain, suggesting that PD1 can down-regulate T cell receptor-mediated signal pathways.

PD1 has two ligands: PD-L1 and PDL2, and they are different in their expression profile. The expression of PDL1 will be up-regulated in macrophages and dendritic cells after treatment with lipopolysaccharide (LPS) and granulocyte-macrophage colony-stimulating factor (GM-CSF), and will also be up-regulated in T cells and B cells after stimulation of T cell receptor and B cell receptor signal pathways. PDL1 is also highly expressed in almost all tumor cells, and the expression will be up-regulated after stimulation of interferon (IFN) gamma. As a matter of fact, the expression of PDL1 in a variety of tumors is considered to have prognostic relevance, but the expression of PD-L2 is relatively concentrated, and mainly on dendritic cells.

When T cells expressing PD-1 come into contact with cells expressing the ligands of PD-1, those antigen-stimulated functional activities, such as cell proliferation, cytokine release and cell lysis activity, are all inhibited. Therefore, the interaction between PD1 and ligands thereof can function as an intrinsic negative feedback regulation mechanism to prevent T cell hyperactivation during infection, immune tolerance or tumorigenesis, thus reducing the occurrence of autoimmune diseases and promoting self tolerance. Long-term antigen stimulation, e.g., in tumor or long-term infection, will cause T cells to express high levels of PD-1, gradually lose activities in response to these long-term antigens, and eventually become nonfunctional, namely, the so-called T cell exhaustion. B cells also have the inhibitory effect caused by PD1 and ligands thereof and corresponding functional exhaustion.

Some evidence from preclinical animal studies has indicated that PD-1 and ligands thereof can down-regulate the immunoreaction. PD-1-deficient mice will develop lupus erythematosus-like acute proliferative glomerulonephritis and dilated cardiomyopathy. Utilizing the antibody of PDL1 to block the interaction between PD-1 and PDL1 has been proved to be able to restore and enhance T cell activation in many systems. The monoclonal antibody of PDL1 can also benefit patients with advanced cancers. In some preclinical animal tumor models, it was also shown that blocking the signal pathway of PD-1/PD-L1 with a monoclonal antibody can enhance immunoreaction and lead to immunoreactions to a series of histologically different tumors. With the long-term infection LCMV model, the interaction between PD-1 and PD-L1 has been found to be able to inhibit the activation and proliferation of virus-specific CD8 T cells and the acquisition of effector cell functions. Besides being capable of enhancing the immunoreaction to long-term antigens, blocking the pathway of PD-1/PDL1 was also found to be able to enhance response to vaccines, including response to a therapeutic vaccine in long-term infection.

To sum up, if, besides the existing monoclonal antibody, a compound for blocking the interaction between PD1 and PDL1 can be developed, it can serve as an effective therapeutic means for blocking the PD-1/PDL1-mediated inhibitory signal pathway to enhance or restore the function of T cells. Therefore, the compound specifically blocking the interaction between PD-1 and PD-L1 will achieve a good therapeutic effect in immunotherapies for a variety of cancers and other immunity-associated diseases.

### SUMMARY

The object of the present invention is to provide biphenyl derivatives for blocking PD-1/ PD-L1 interaction, and thus to develop a new generation of PD-1/PD-L1 inhibitors.

The first aspect of the present invention provides a compound of formula (I), a stereoisomer, prodrug or pharmaceutically acceptable salt thereof: 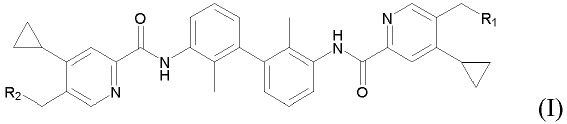 wherein,
R₁ is selected from the group consisting of: 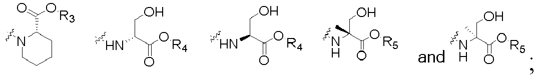
R₂ is -NHR₆ or 4-6 membered nitrogen-containing heterocyclyl, wherein a nitrogen atom is linked to methylene, and the 4-6 membered nitrogen-containing heterocyclyl is optionally further substituted with one or more substituents selected from the group consisting of deuterium, halogen, cyano, C₁₋₄ alkyl, C₁₋₄ haloalkyl, hydroxy-substituted C₁₋₄ alkyl, C₁₋₄ deuterioalkyl, C₃₋₆ cycloalkyl, 4-6 membered heterocyclyl, hydroxy-substituted C₃₋₆ cycloalkyl, carboxy-substituted C₃₋₆ cycloalkyl, hydroxy-substituted 4-6 membered heterocyclyl, carboxy-substituted 4-6 membered heterocyclyl, hydroxy, C₁₋₄ alkoxy, carboxy and C₁₋₄ alkoxyacyl;
R₃, R₄ and R₅ are each independently selected from the group consisting of hydrogen, deuterium and C₁₋₄ alkyl, the C₁₋₄ alkyl is optionally further substituted with one or more substituents selected from the group consisting of deuterium, halogen, cyano, cyclopropyl, hydroxy and C₁₋₄ alkoxy;
R₆ is selected from the group consisting of C₁₋₄ alkyl, C₃₋₆ cycloalkyl and C₄₋₆ heterocyclyl, the heterocyclyl optionally contains 1-2 heteroatoms selected from N and O, above groups are optionally further substituted with one or more substituents selected from the group consisting of deuterium, halogen, cyano, C₁₋₄ alkyl, C₁₋₄ haloalkyl, hydroxy-substituted C₁₋₄ alkyl, C₁₋₄ deuterioalkyl, C₃₋₆ cycloalkyl, 4-6 membered heterocyclyl, hydroxy-substituted C₃₋₆ cycloalkyl, carboxy-substituted C₃₋₆ cycloalkyl, hydroxy-substituted 4-6 membered heterocyclyl, carboxy-substituted 4-6 membered heterocyclyl, hydroxy, C₁₋₄ alkoxy, carboxy and C₁₋₄ alkoxyacyl;
R₂ is the same as or different from R₁, provided that when R₁ is 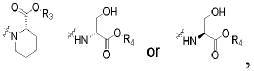 R₂ is different from R₁, and when R₁ is 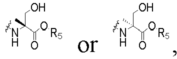 R₂ is the same as or different from R₁.

As a preferred embodiment, in the compound of formula (I), the stereoisomer, prodrug or pharmaceutically acceptable salt thereof, R₃, R₄ and R₅ are each independently selected from the group consisting of hydrogen, deuterium, methyl, ethyl and propyl, above groups are optionally further substituted with one or more substituents selected from the group consisting of deuterium, fluoro, chloro, bromo, cyano, cyclopropyl, hydroxy, methoxy and ethoxy;
R₆ is selected from the group consisting of methyl, ethyl, isopropyl, cyclopropyl, cyclobutyl, cyclopentyl, cyclohexyl, 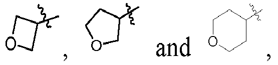 above groups are optionally further substituted with one or more substituents selected from the group consisting of deuterium, fluoro, chloro, bromo, cyano, nitro, azido, methyl, ethyl, isopropyl, difluoromethyl, trifluoromethyl, dichloromethyl, trichloromethyl, dibromomethyl, tribromomethyl, dideuteriomethyl, trideuteriomethyl, hydroxymethyl, cyclopropyl, cyclobutyl, cyclopentyl, hydroxycyclopropyl, 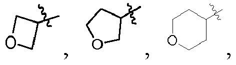 hydroxy, methoxy, ethoxy, carboxy, methoxyacyl and ethoxyacyl.

As a preferred embodiment, in the compound of formula (I), the stereoisomer, prodrug or pharmaceutically acceptable salt thereof, the compound of formula (I) is a compound having formula (II a), formula (II b), formula (II c), formula (II d) or formula (II e): 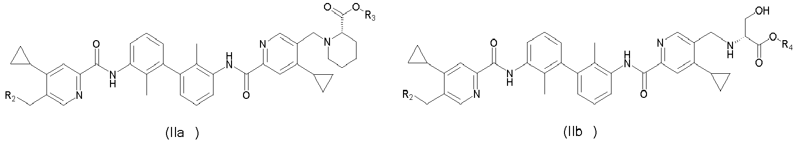 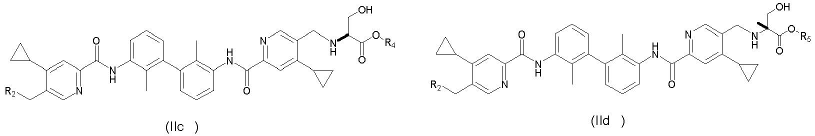 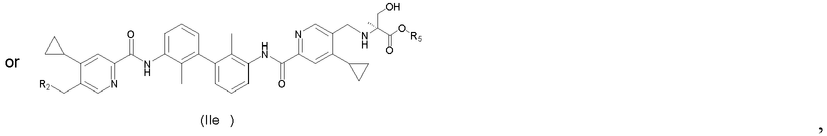 wherein R₂ is -NHR₆ or 4-6 membered nitrogen-containing heterocyclyl, wherein a nitrogen atom is linked to methylene, provided that R₂ in the compound of formula (IIa) is not 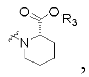 R₂ in the compound of formula (IIb) is not 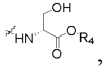 R₂ in the compound of formula (IIc) is not 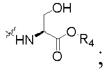 the 4-6 membered nitrogen-containing heterocyclyl is selected from the following structures: 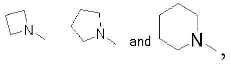 above groups are optionally further substituted with one or more substituents selected from the group consisting of deuterium, fluoro, chloro, bromo, cyano, methyl, ethyl, isopropyl, cyclopropyl, hydroxycyclopropyl, hydroxymethyl, hydroxy, methoxy, ethoxy, carboxy, methoxyacyl and ethoxyacyl;
R₃, R₄ and R₅ are each independently selected from the group consisting of hydrogen, deuterium, methyl and ethyl;
R₆ is selected from the group consisting of methyl, ethyl, isopropyl, cyclopropyl, cyclobutyl, cyclopentyl, cyclohexyl, 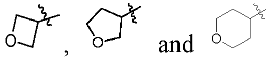 , above groups are optionally further substituted with one or more substituents selected from the group consisting of deuterium, fluoro, chloro, bromo, cyano, methyl, ethyl, isopropyl, cyclopropyl, hydroxycyclopropyl, hydroxymethyl, hydroxy, methoxy, ethoxy, carboxy, methoxyacyl and ethoxyacyl.

As a preferred embodiment, the compound of formula (I), the stereoisomer, prodrug or pharmaceutically acceptable salt thereof is selected from the following compounds:

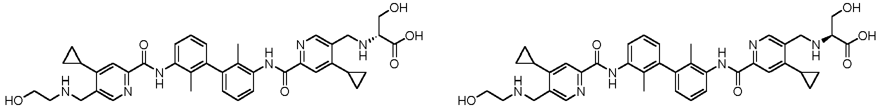

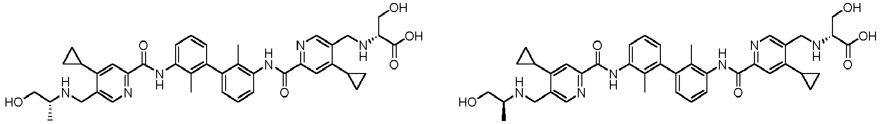

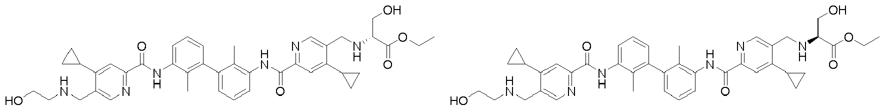

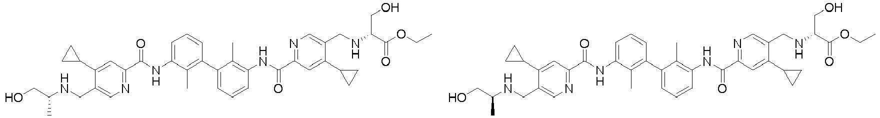

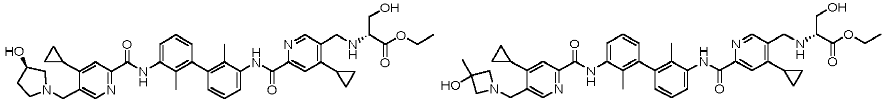

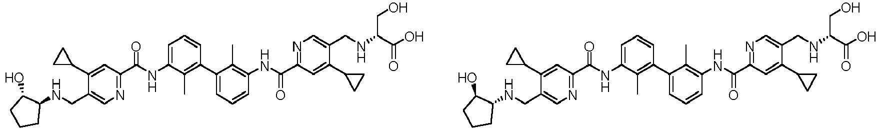

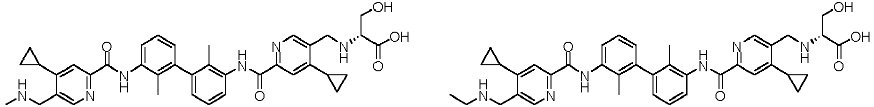

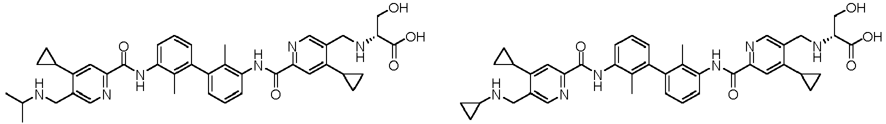

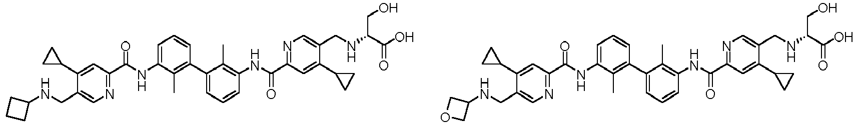

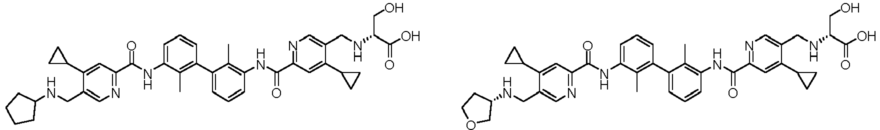

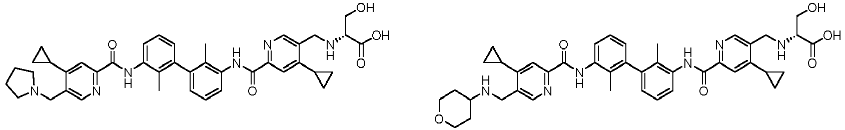

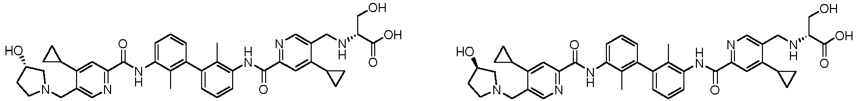

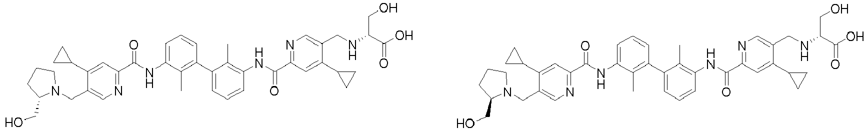

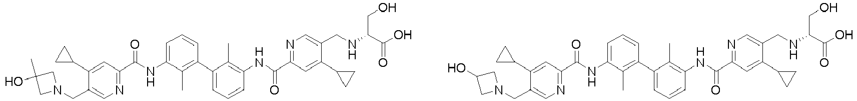

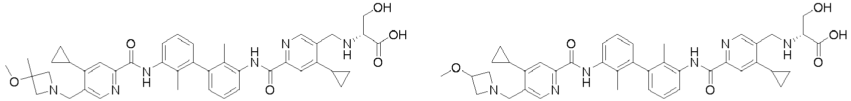

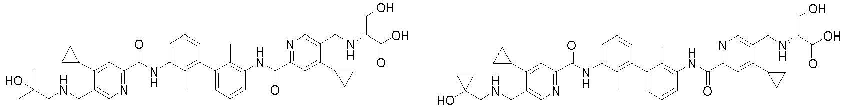

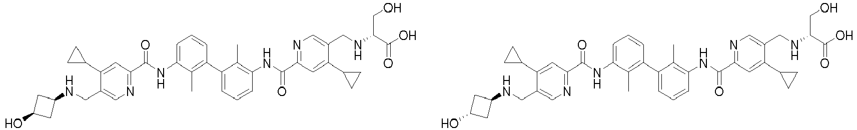

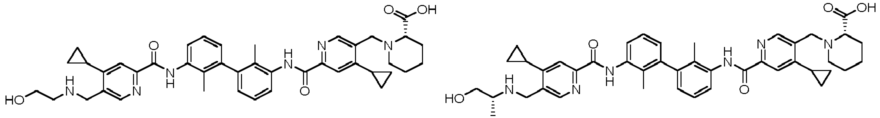

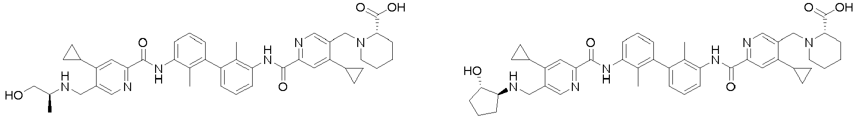

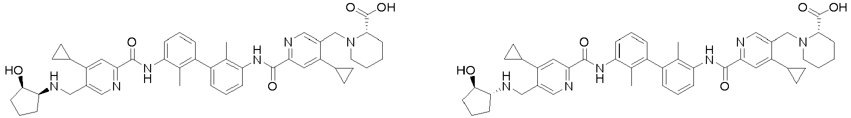

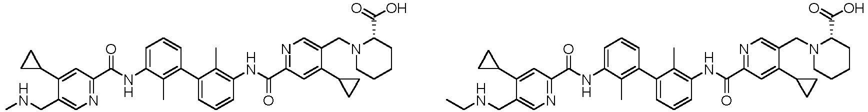

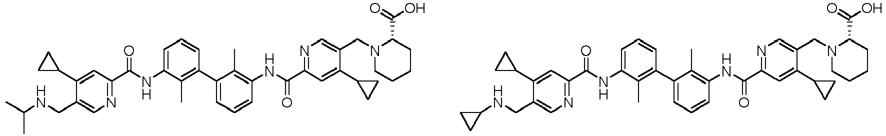

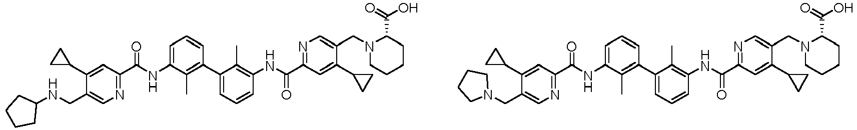

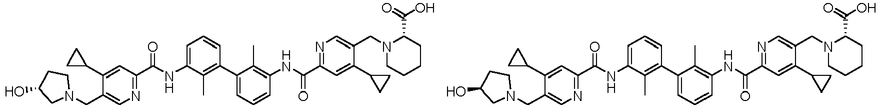

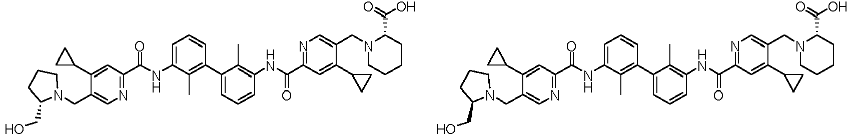

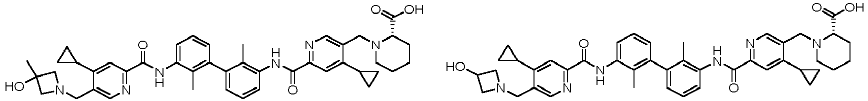

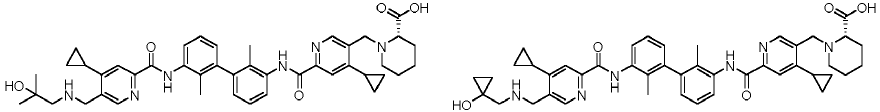

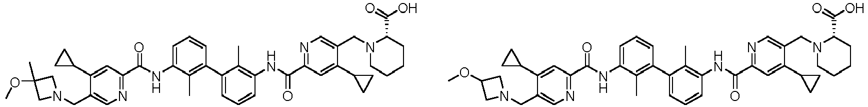

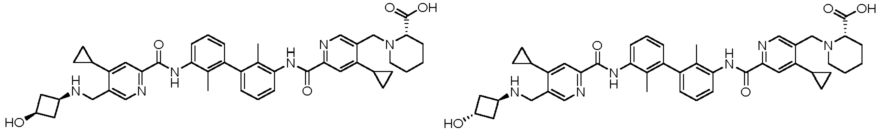

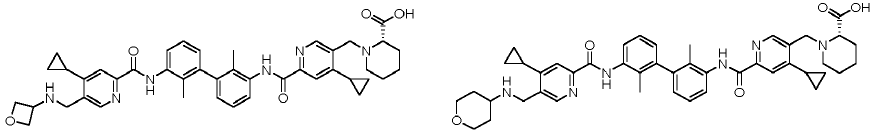

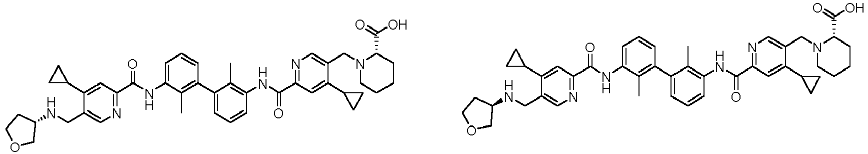

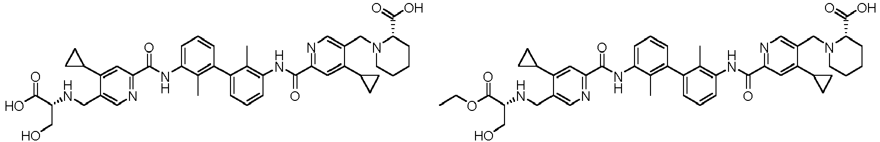

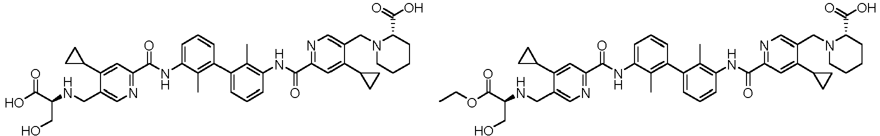

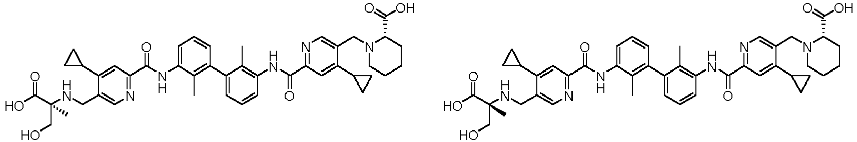

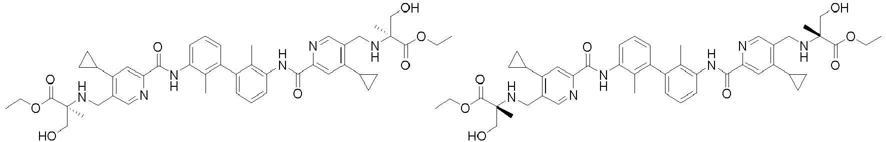

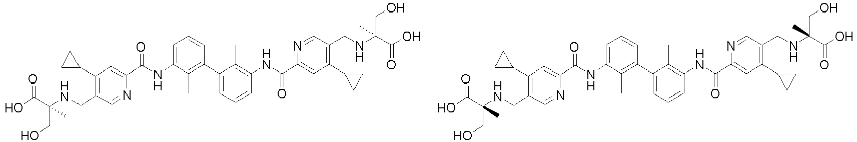

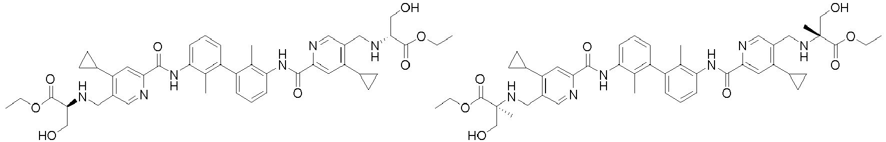

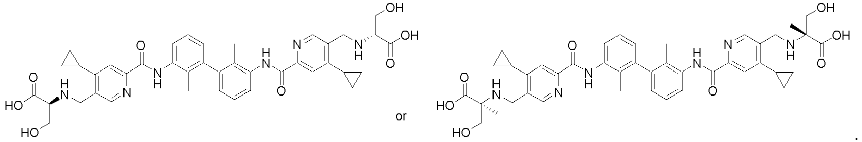

The second aspect of the present invention provides a method for preparing the aforementioned compound of formula (I), the stereoisomer, prodrug or pharmaceutically acceptable salt thereof, comprising the following steps: 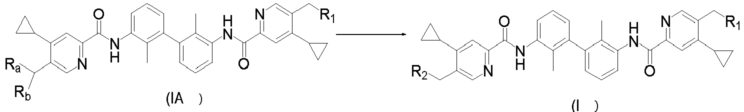 or, 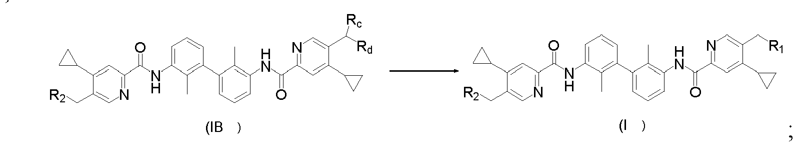 wherein, Rₐ, R_{b}, R_{c} and R_{d} are each independently C₁₋₈ alkoxy, or Rₐ together with R_{b}, R_{c} together with R_{d} is =O, and R₁ and R₂ are defined as those in the compound of formula (I).

The third aspect of the present invention provides a pharmaceutical composition, comprising the aforementioned compound of formula (I), the stereoisomer, prodrug or pharmaceutically acceptable salt thereof, and pharmaceutically acceptable carrier.

The fourth aspect of the present invention provides uses of the aforementioned compound of formula (I), the stereoisomer, prodrug or pharmaceutically acceptable salt thereof in the preparation of a medicament for preventing and/or treating a PD-1/PD-L1 signal pathway-mediated disease.

As a preferred embodiment, the aforementioned PD-1/PD-L1 signal pathway-mediated disease is cancer or tumor, immune-related disease and disorder, communicable disease, infectious disease or metabolic disease.

As a further preferred embodiment, the infectious disease is bacterial infectious disease, viral infectious disease and fungal infectious disease; the cancer or tumor islymphoma (including but not limited to lymphocytic lymphoma, primary central nervous system lymphoma, T cell lymphoma, diffuse large B cell lymphoma, follicle center lymphoma, Hodgkin lymphoma, non-Hodgkin lymphoma or primary mediastinal large B cell lymphoma), sarcoma (including but not limited to Kaposi's sarcoma, fibrosarcoma, liposarcoma, chondrosarcoma, osteosarcoma, leiomyosarcoma, rhabdomyosarcoma, soft tissue sarcoma, angiosarcoma or lymphangiosarcoma), melanoma, glioblastoma, synovioma, meningioma, biliary tract tumor, thymic tumor, neuroma, seminoma, nephroblastoma, pleomorphic adenoma, hepatocellular papilloma, renal tubule adenoma, cystadenoma, papilloma, adenoma, leiomyoma, rhabdomyoma, hemangioma, lymphangioma, osteoma, chondroma, lipoma, fibroma, central nervous system tumor, rhachiophyma, brain stem glioma, pituitary adenoma, multiple myeloma, ovarian tumor, myelodysplastic syndrome or mesothelioma, prostate cancer, recurrent prostate cancer or prostate cancer having developed resistance to existing medicaments, thyroid cancer, parathyroid cancer, anal cancer, testicular cancer, urethral carcinoma, penile cancer, bladder cancer, ureteral cancer, uterine cancer, ovarian cancer, fallopian tube cancer, endometrial cancer, cervical cancer, vaginal cancer, vulvar cancer, adrenal cancer, Merkel cell carcinoma, embryonal carcinoma, chronic or acute leukemia (including but not limited to acute myeloid leukemia, chronic myeloid leukemia, acute lymphoblastic leukemia, chronic granulocytic leukemia and chronic lymphoblastic leukemia), bronchial carcinoma, esophageal cancer, nasopharyngeal carcinoma, hepatocellular carcinoma, renal cell carcinoma, small cell lung cancer, basal cell carcinoma, lung cancer, breast cancer, adenocarcinoma, papillary carcinoma, cystadenocarcinoma, squamous non-small cell lung cancer, non-squamous non-small cell lung cancer, rectal cancer, colon cancer, colorectal cancer, gastric cancer, pancreatic cancer, head and neck squamous cell carcinoma, head and neck cancer, gastrointestinal cancer, bone cancer, skin cancer, small intestine cancer, endocrine system cancer, renal pelvic carcinoma, epidermoid carcinoma, abdominal wall carcinoma, renal cell carcinoma, transitional cell carcinoma, choriocarcinoma, or metastatic tumor, especially metastatic tumor expressing PD-L1;
the immune-related disease and disorder is rheumatic arthritis, renal failure, lupus erythematosus, asthma, psoriasis, ulcerative colitis, pancreatitis, allergy, fibrosis, anemia, fibromyalgia, Alzheimer's disease, congestive heart failure, stroke, aortic valve stenosis, arteriosclerosis, osteoporosis, Parkinson's disease, infection, Crohn's disease, ulcerative colitis, allergic contact dermatitis and eczema, systemic sclerosis or multiple sclerosis;
the communicable disease or infectious disease is sepsis, liver infection, HIV, hepatitis A, hepatitis B, hepatitis C, hepatitis D, herpes virus, papillomavirus or influenza;
the metabolic disease is diabetes, diabetic ketoacidosis, hyperglycemic hyperosmolar syndrome, hypoglycemia, gout, malnutrition, vitamin A deficiency, scurvy, vitamin D deficiency or osteoporosis.

The fifth aspect of the present invention provides the aforementioned compound of formula (I), the stereoisomer, prodrug or pharmaceutically acceptable salt thereof for use as a medicament for preventing and/or treating PD-1/PD-L1 signal pathway-mediated cancer or tumor, immune-related disease and disorder, communicable disease, infectious disease or metabolic disease.

### DETAILED DESCRIPTION OF THE INVENTION

After extensive and intensive research, the inventors of the present invention, for the first time, developed a biphenyl series of compounds having the structure of the general formula (I). With a strong inhibitory effect on the interaction between PD-1 and PD-L1, the series of compounds of the present invention can be widely applied in the preparation of medicaments for preventing and/or treating cancer or tumor, immune-related disease and disorder, communicable disease, infectious disease or metabolic disease that is mediated by the PD-1/PD-L1 signal pathway, and are expected to be developed into a new generation of PD-1/PD-L1 inhibitors. The present invention is achieved on this basis.

Detailed description: unless otherwise stated or specified, the following terms used in the specification and claims have the following meanings.

"Alkyl" refers to linear or branched saturated aliphatic alkyl groups, for example, "C₁₋₄ alkyl" refers to straight and branched chain alkyl groups comprising 1 to 4 carbon atoms, including but not limited to methyl, ethyl, *n*-propyl, isopropyl, *n*-butyl, isobutyl, *t*-butyl, *sec*-butyl, and the like. Alkyl may be optionally substituted or unsubstituted, and when it is substituted, the substituent is preferably one or more (preferably, 1, 2, 3 or 4) of the groups independently selected from the group consisting of deuterium, halogen, cyano, C₁₋₄ alkyl, C₁₋₄ haloalkyl, hydroxy-substituted C₁₋₄ alkyl, C₁₋₄ deuterioalkyl, C₃₋₆ cycloalkyl, 4-6 membered heterocyclyl, hydroxy-substituted C₃₋₆ cycloalkyl, carboxy-substituted C₃₋₆ cycloalkyl, hydroxy-substituted 4-6 membered heterocyclyl, carboxy-substituted 4-6 membered heterocyclyl, hydroxy, C₁₋₄ alkoxy, carboxy and C₁₋₄ alkoxyacyl.

"Cycloalkyl" refers to monocyclic or polycyclic hydrocarbon substituents that are saturated or partially unsaturated, for example, "C₃₋₆ cycloalkyl" refers to monocyclic cycloalkyl containing 3 to 6 carbon atoms, including but not limited to cyclopropyl, cyclobutyl, cyclopentyl, cyclopentenyl, cyclohexyl, cyclohexenyl, cyclohexadienyl and the like. Cycloalkyl may be optionally substituted or unsubstituted, and when it is substituted, the substituent is preferably one or more (preferably, 1, 2, 3 or 4) of the groups independently selected from the group consisting of deuterium, halogen, cyano, C₁₋₄ alkyl, C₁₋₄ haloalkyl, hydroxy-substituted C₁₋₄ alkyl, C₁₋₄ deuterioalkyl, C₃₋₆ cycloalkyl, 4-6 membered heterocyclyl, hydroxy-substituted C₃₋₆ cycloalkyl, carboxy-substituted C₃₋₆ cycloalkyl, hydroxy-substituted 4-6 membered heterocyclyl, carboxy-substituted 4-6 membered heterocyclyl, hydroxy, C₁₋₄ alkoxy, carboxy and C₁₋₄ alkoxyacyl.

"Heterocyclyl" refers to a monocyclic hydrocarbon substituent that is saturated or partially unsaturated, wherein 1 or 2 (preferably, 1) ring atoms are heteroatoms selected from the group consisiting of nitrogen, oxygen and S(O)ᵣ (wherein r is an integer of 0, 1 or 2), excluding ring portions of -O-O-, -O-S- or -S-S-, and the remaining ring atoms are carbon atoms. For example, "4-6 membered heterocyclyl" refers to monocyclic heterocyclyl containing 4 to 6 ring atoms, including but not limited to pyrrolidinyl, piperidinyl, piperazinyl, morpholinyl, thiomorpholinyl, homopiperazinyl, and the like. Heterocyclyl may be optionally substituted or unsubstituted, and when it is substituted, the substituent is preferably one or more (preferably, 1, 2, 3 or 4) of the groups independently selected from the group consisting of deuterium, halogen, cyano, C₁₋₄ alkyl, C₁₋₄ haloalkyl, hydroxy-substituted C₁₋₄ alkyl, C₁₋₄ deuterioalkyl, C₃₋₆ cycloalkyl, 4-6 membered heterocyclyl, hydroxy-substituted C₃₋₆ cycloalkyl, carboxy-substituted C₃₋₆ cycloalkyl, hydroxy-substituted 4-6 membered heterocyclyl, carboxy-substituted 4-6 membered heterocyclyl, hydroxy, C₁₋₄ alkoxy, carboxy and C₁₋₄ alkoxyacyl.

"Alkoxy" refers to -O-(alkyl), wherein the alkyl is defined as above, for example, "C₁₋₄ alkoxy" refers to an alkoxy containing 1 to 4 carbons atoms, including but not limited to methoxy, ethoxy, propoxy, butoxy, and the like. Alkoxy may be optionally substituted or unsubstituted, and when it is substituted, the substituent is preferably one or more (preferably, 1, 2, 3 or 4) of the groups independently selected from the group consisting of deuterium, halogen, cyano, C₁₋₄ alkyl, C₁₋₄ haloalkyl, hydroxy-substituted C₁₋₄ alkyl, C₁₋₄ deuterioalkyl, C₃₋₆ cycloalkyl, 4-6 membered heterocyclyl, hydroxy-substituted C₃₋₆ cycloalkyl, carboxy-substituted C₃₋₆ cycloalkyl, hydroxy-substituted 4-6 membered heterocyclyl, carboxy-substituted 4-6 membered heterocyclyl, hydroxy, C₁₋₄ alkoxy, carboxy and C₁₋₄ alkoxyacyl.

"C₁₋₄ haloalkyl" refers to an alkyl having 1 to 4 carbon atoms in which hydrogens on the alkyl are optionally substituted with a fluorine, chlorine, bromine or iodine atom, including but not limited to difluoromethyl, dichloromethyl, dibromomethyl, trifluoromethyl, trichloromethyl, tribromomethyl, and the like.

"C₁₋₄ alkoxyacyl" refers to C₁₋₄ alkoxy being linked to the carbonyl [C(O)], and is also generally referred to as "C₁₋₄ alkyl-O-C(O)-", for example, C₁ alkoxyacyl may be referred to as "C₁ alkyl-O-C(O)-", and refers to methoxyacyl; C₂ alkoxyacyl may be referred to as "C₂ alkyl-O-C(O)-", and refers to ethoxyacyl. "Halogen" refers to fluorine, chlorine, bromine or iodine. "PE" refers to petroleum ether, "EtOAc"/"EA" refers to ethyl acetate, "MeOH" refers to methanol, "DMF" refers to *N*,*N*-dimethylformamide, "THF" refers to tetrahydrofuran, "DIPEA" refers to *N*,*N*-diisopropylethylamine, "HATU" refers to 2-(7-azabenzotriazol-1-yl)-*N*,*N*,*N*',*N*'-tetramethyluronium hexafluorophosphate, "NaHCO₃" refers to sodium bicarbonate, "KMnO₄" refers to potassium permanganate, and "NaBH₃CN" refers to sodium cyanoborohydride.

The term "optional" or "optionally" means that the event or circumstance subsequently described may, but not necessarily, occur, and that the description includes instances where the event or circumstance occurs or does not occur, that is, instances where substitution occurs or does not occur. For example, "heterocyclyl group optionally substituted by alkyl" means that alkyl may be, but not necessarily, present, and that the description includes instances where the heterocyclyl group is or is not substituted by alkyl.

The term "substituted" means that one or more hydrogen atoms in the group are each independently substituted by a corresponding number of substituents. It goes without saying that a substituent is only in its possible chemical position and consistent with Chemical Valence Bond Theory, and those skilled in the art will be able to determine (by experiments or theories) possible or impossible substitution without undue efforts. For example, it may be unstable when an amino or hydroxy having a free hydrogen is bound to a carbon atom having an unsaturated bond (such as olefin).

"Stereoisomers" refer to isomers produced by different spatial arrangements of atoms in molecules, and can be classified into *cis-trans* isomers and enantiomers, and also into enantiomers and diastereomers. Stereoisomers resulting from rotation of single bonds are referred to as conformational stereo-isomers and sometimes also as rotamers. Stereoisomers resulting from bond lengths, bond angles, intramolecular double bonds, rings and the like are referred to as configuration stereo-isomers, and the configuration stereo-isomers are classified into two categories. Among them, isomers resulting from the fact that a double bond or a single bond of a ring-forming carbon atom cannot rotate freely are referred to as geometric isomers and also as *cis-trans* isomers, and the isomers are classified into Z, E configurations. For example, *cis*-2-butene and *trans*-2-butene are a pair of geometric isomers, and stereoisomers having different optical rotation properties due to the absence of anti-axisymmetry in the molecule are referred to as optical isomers, and are classified into R, S configurations. In the present invention, the term "stereoisomer" is understood to include one or more of the above enantiomers, configuration isomers and conformational isomers, unless otherwise specified.

"Pharmaceutically acceptable salts" as used herein refers to pharmaceutically acceptable acid addition salts, including inorganic and organic acid salts, which may be prepared by methods known in the art.

"Pharmaceutical composition" refers to a mixture containing one or more of the compounds described herein or a physiologically/pharmaceutically acceptable salt or pro-drug thereof, and other chemical components, for example physiologically/pharmaceutically acceptable carriers and excipients. The purpose of the pharmaceutical composition is to promote the administration to an organism, which facilitates the absorption of the active ingredient, thereby exerting biological activities.

**The present invention is further explained in detail below with reference to examples, which are not intended to limit the present invention, and the present invention is not merely limited to the contents of the examples.**

The compound structure of the present invention is determined by nuclear magnetic resonance (NMR) and/or liquid chromatography-mass spectrometry (LC-MS). The NMR chemical shift (δ) is given in parts per million (ppm). The NMR determination is conducted by using a Bruker AVANCE-400 or an AVANCE-500 nuclear magnetic resonance apparatus, with deuterated dimethyl sulfoxide (DMSO-*d₆*), deuterated methanol (CD₃OD), and deuterium oxide (D₂O) and deuterated chloroform (CDCl₃) as determination solvents, and tetramethylsilane (TMS) as internal standard.

The LC-MS determination is conducted by using an Agilent 6120 mass spectrometer. The HPLC determination is conducted by using an Agilent 1200 DAD high pressure liquid chromatograph (Sunfire C18 150 ^{∗} 4.6 mm chromatographic column) and a Waters 2695-2996 high pressure liquid chromatograph (Gimini C18 150 ^{∗} 4.6 mm chromatographic column).

Yantai Yellow Sea HSGF254 or Qingdao GF254 silica gel plate is adopted as a thin layer chromatography (TLC) silica gel plate. The specification adopted by the TLC is 0.15-0.20 mm, and the specification adopted by the thin layer chromatography for the separation and purification of products is 0.4-0.5 mm. The Yantai Yellow Sea silica gel of 200-300 mesh is generally utilized as a carrier in column chromatography.

Starting materials in the examples of the present invention are known and commercially available, or may be synthesized by using or according to methods known in the art.

Unless otherwise stated, all reactions of the present invention are carried out under a dry nitrogen or argon atmosphere with continuous magnetic stirring, wherein the solvent is a dry solvent, and the reaction temperature is in degree centigrade (°C).

### Preparation of Examples

### Example 1: Preparation of (S)-1-((4-cyclopropyl-6-((3'-(4-cyclopropyl-5-(((2-hydroxyeth yl)amino)methyl)picolinamido)-2,2'-dimethyl-[1,1'-biphenyl]-3-yl)carbamoyl) pyridin-3-yl)methyl)piperidine-2-carboxylic acid

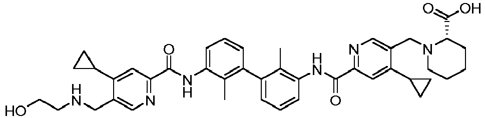

### Step 1: Synthesis of 4-cyclopropyl-5-(dimethoxymethyl)-2-vinylpyridine

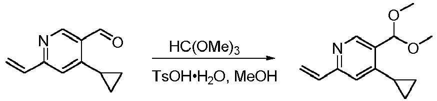

4-cyclopropyl-6-vinyl-3-nicotinaldehyde (11.1 g, 64.0 mmol), trimethyl orthoformate (40.7 g, 384 mmol) and *p*-toluenesulfonic acid monohydrate (6.08 g, 32.0 mmol) were weighed and dissolved in absolute methanol (115 mL). The mixture were heated to 55 °C and stirred overnight. The reaction mixture was neutralized by adding an appropriate amount of DIPEA, and directly concentrated to obtain a crude product. The crude product was separated by silica gel column chromatography (PE/EA: 0-5%) to obtain 4-cyclopropyl-5-(dimethoxymethyl)-2-vinylpyridine (11.1 g, yield: 79%). MS m/z (ESI): 220.2 [M+H]⁺.

### Step 2: Synthesis of potassium 4-cyclopropyl-5-(dimethoxymethyl)picolinate

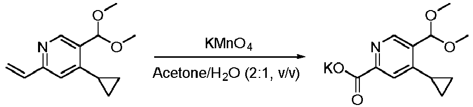

4-cyclopropyl-5-(dimethoxymethyl)-2-vinylpyridine (4.0 g, 23.1 mmol) was dissolved in a mixture of acetone/water (60 mL, v/v = 2:1) and KMnO₄ (8.56 g, 69.3 mmol) was added in batches. The reaction solution was stirred at room temperature overnight and heated to 40 °C and then reacted for 6 hrs. After the reaction was completed, the resulting reaction mixture was filtered, and washed with MeOH/CH₃CN (50 mL, v/v = 1:1). The filtrate was combined, added with active carbon, heated to 40 °C and stirred for 0.5 hrs, filtered while hot, and washed by adding H₂O (20 mL). The filtrate was concentrated under reduced pressure to remove most of the organic solvent, and the remaining aqueous phase was lyophilized to obtain potassium 4-cyclopropyl-5-(dimethoxymethyl)picolinate (5.1 g, crude).

### Step 3: Synthesis of 4-cyclopropyl-5-formylpicolinic acid

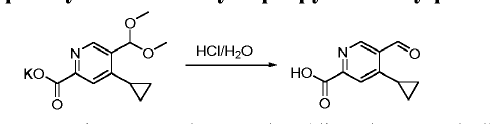

Potassium 4-cyclopropyl-5-(dimethoxymethyl)picolinate (2.7 g, 9.8 mmol) was dissolved in pure water (20 mL), the reaction solution was added with a hydrochloric acid solution (10 L, 4.0 M), and stirred at room temperature for about 2 hrs. After the reaction was completed, the reaction solution was lyophilized to obtain 4-cyclopropyl-5-formylpicolinic acid (2.2 g, crude). MS m/z (ESI): 189.8 [M-H]⁻.

### Step 4: Synthesis of 4-cyclopropyl-N-(3'-(4-cyclopropyl-5-(dimethoxymethyl)picolinamido)-2,2'-dimethyl-[1,1'-biphenyl]-3-yl)-5-formylpicolinamide

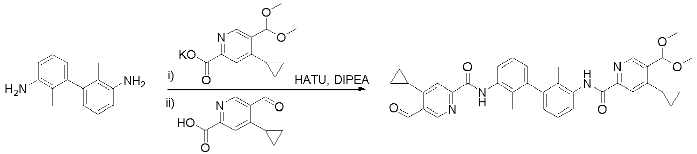

Potassium 4-cyclopropyl-5-(dimethoxymethyl)picolinate (622 mg, 2.27 mmol), HATU (854 mg, 2.06 mmol), 2,2'-dimethyl-[1,1'-diphenyl]-3,3'-diamine (435 mg, 2.05 mmol) an d DIPEA (1.0 mL) were weighed into a 100 mL eggplant-shaped flask, THF/MTBE (12 mL, 1:1) was added, and the mixture was stirred at room temperature overnight. The r eaction solution was added with 4-cyclopropyl-5-formylpicolinic acid (452 mg, 2.0 mmo 1), HATU (598 mg, 1.57 mmol), and DMF (1.0 mL), and stirred at room temperature fo r 3 hrs. The reaction solution was added with water (20 mL) and extracted 2 times wit h EtOAc (50 mL), and the organic phase was washed with brine (10 mL). The reaction solution was dried over anhydrous Na₂SO₄, filtered and concentrated to obtain a crude product. The crude product was separated by silica gel column chromatography (PE/EA: 0-12%) to obtain 4-cyclopropyl-*N*-(3'-(4-cyclopropyl-5-(dimethoxymethyl)picolinamido)-2,2'-dimethyl-[1,1'-biphenyl]-3-yl)-5-formylpicolinamide (344 mg, yield: 28%). MS m/z (ESI): 651.6 [M+H]⁺.

### Step 5: Synthesis of (S)-1-((4-cyclopropyl-6-((3'-(4-cyclopropyl-5-formylpicolinamido)-2,2'-dimethyl-[1,1'-biph enyl]-3-yl)carbamoyl)pyridin-3-yl)methyl)piperidine-2-carboxylic acid

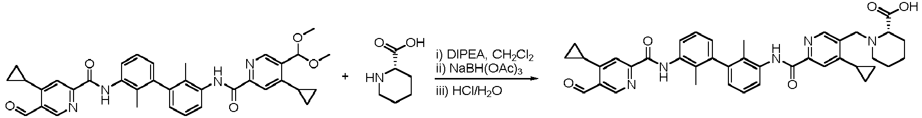

4-cyclopropyl-*N*-(3'-(4-cyclopropyl-5-(dimethoxymethyl)picolinamido)-2,2'-dimethyl-[1,1'-b iphenyl]-3-yl)-5-formylpicolinamide (266 mg, 0.439 mmol) and (*S*)-piperidine-2-carboxylic acid (227 mg, 1.756 mmol) were added to a mixture solvent of CH₂Cl₂/DIPEA (20 mL, 4:1), and the mixture was heated to reflux and stirred for 22 hrs. The reaction solution was added with NaBH(OAc)₃ (372.3 mg, 1.756 mmol), stirred and reacted for 2 hrs at reflux. After the reaction was completed, the reaction solution was separated by reversed-phase column chromatography, and the collected resulting solution was concentrated to remove the organic solvent, added with a hydrochloric acid solution (4.0 mL, 4 M) and stirred for 2 hrs. The reaction solution was lyophilized to obtain (*S*)-1-((4-cyclopropyl-6-((3'-(4-cyclopropyl-5-formylpicolinamido)-2,2'-dimethyl-[1,1'-biphenyl ]-3-yl)carbamoyl)pyridin-3-yl)methyl)piperidine-2-carboxylic acid (153 mg, yield: 52%). MS m/z (ESI): 670.2 [M-H]⁻.

### Step 6: Synthesis of (S)-1-((4-cyclopropyl-6-((3'-(4-cyclopropyl-5-(((2-hydroxyethyl)amin o)methyl)picolinamido)-2,2'-dimethyl-[1,1'-biphenyl]-3-yl)carbamoyl)pyridin-3-yl)meth yl)piperidine-2-carboxylic acid

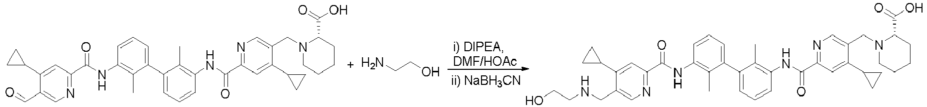

(*S*)-1-((4-cyclopropyl-6-((3'-(4-cyclopropyl-5-formylpicolinamido)-2,2'-dimethyl-[1,1'-biphenyl]-3-yl)carbamoyl)pyridin-3-yl)methyl)piperidine-2-carboxylic acid (48.0 mg, 0.071 mmol), ethanolamine (22.0 mg, 0.357 mmol) and DIPEA (0.1 mL, 0.43 mmol) were dis solved in DMF/HOAc (3 mL, 5:1), and the reaction solution was stirred at room temper ature for 2 hrs. The reaction solution was added with NaBH₃CN (22.4 mg, 0.355 mmo 1), and stirred at room temperature for 1 hr. After the reaction was completed, 3 drops of methanol was added to terminate the reaction. The crude product was separated by re versed-phase column chromatography to obtain (*S*)-1-((4-cyclopropyl-6-((3'-(4-cyclopropyl-5-(((2-hydroxyethyl)amino)methyl)picolinamido)-2,2'-dimethyl-[1,1'-biphenyl]-3-yl)carbamoyl)pyri din-3-yl)methyl)piperidine-2-carboxylic acid (26.5 mg, yield: 52%). MS m/z (ESI): 715.4 [M-H]⁻.

¹H NMR (400 MHz, DMSO-*d*₆) δ 10.33 (s, 1H), 10.31 (s, 1H), 8.62 (s, 1H), 8.52 (s, 1H), 7.86 (t, *J* = 7.9 Hz, 2H), 7.61 (s, 1H), 7.59 (s, 1H), 7.32 (t, *J* = 7.8 Hz, 2H), 6.98 (dd, *J* = 7.6, 4.5 Hz, 2H), 4.17 (s, 2H), 4.03 (d, *J* = 13.7 Hz, 1H), 3.71 (d, *J* = 13.6 Hz, 1H), 3.58 (t, *J* = 5.7 Hz, 2H), 3.19 (t, *J* = 5.6 Hz, 1H), 2.90 - 2.79 (m, 2H), 2.33 - 2.19 (m, 2H), 2.00 (s, 6H), 1.84 - 1.72 (m, 2H), 1.55 - 1.36 (m, 4H), 1.24 (d, *J* = 6.7 Hz, 2H), 1.20 - 1.06 (m, 4H), 0.95 - 0.85 (m, 4H).

Examples 2 to 28 were prepared by selecting corresponding raw materials according to all or part of the synthesis method in Example 1.

| Example No. | Structure | Name | MS: *m*/*z* [M+1]⁺ |
|---|---|---|---|
| 2 | 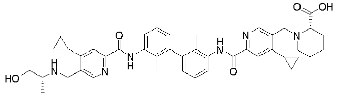 | (*S*)-1-((4-cyclopropyl-6-((3'-(4-cyclo propyl-5-((((*R*)-1-hydroxypropan-2-y l)amino)methyl)picolinamido)-2,2'-di methyl-[1,1'-biphenyl]-3-yl)carbamo yl)pyridin-3-yl)methyl)piperidine-2-c arboxylic acid | 731.6 |
| 3 | 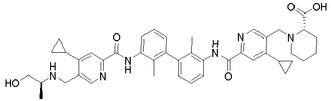 | (*S*)-1-((4-cyclopropyl-6-((3'-(4-cyclo propyl-5-((((*S*)-1-hydroxypropan-2-y l)amino)methyl)picolinamido)-2,2'-di methyl-[1,1'-biphenyl]-3-yl)carbamo yl)pyridin-3-yl)methyl)piperidine-2-c arboxylic acid | 731.6 |
| 4 | 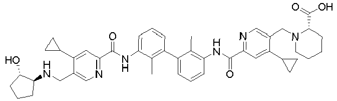 | (*S*)-1-((4-cyclopropyl-6-((3'-(4-cyclo propyl-5-((((1*S*,2*S*)-2-hydroxycyclop entyl)amino)methyl)picolinamido)-2, 2'-dimethyl-[1,1'-biphenyl]-3-yl)carb amoyl)pyridin-3-yl)methyl)piperidine -2-carboxylic acid | 757.6 |
| 5 | 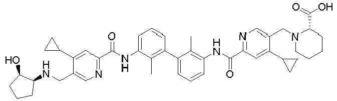 | (*S*)-1-((4-cyclopropyl-6-((3'-(4-cyclo propyl-5-((((1*S*,2*R*)-2-hydroxycyclop entyl)amino)methyl)picolinamido)-2, 2'-dimethyl-[1,1'-biphenyl]-3-yl)carb amoyl)pyridin-3-yl)methyl)piperidine -2-carboxylic acid | 757.6 |
| 6 | 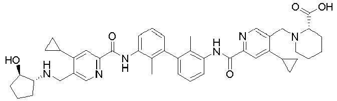 | (*S*)-1-((4-cyclopropyl-6-((3'-(4-cyclo propyl-5-((((1*R*,2*R*)-2-hydroxycyclop entyl)amino)methyl)picolinamido)-2, 2'-dimethyl-[1,1'-biphenyl]-3-yl)carb amoyl)pyridin-3-yl)methyl)piperidine -2-carboxylic acid | 757.6 |
| 7 | 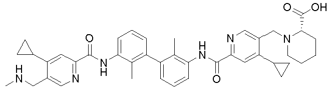 | (*S*)-1-((4-cyclopropyl-6-((3'-(4-cyclo propyl-5-((methylamino)methyl)picol inamido)-2,2'-dimethyl-[1,1'-bipheny l]-3-yl)carbamoyl)pyridin-3-yl)methy l)piperidine-2-carboxylic acid | 687.4 |
| 8 | 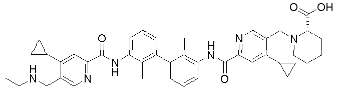 | (*S*)-1-((4-cyclopropyl-6-((3'-(4-cyclo propyl-5-((ethylamino)methyl)picolin amido)-2,2'-dimethyl-[1,1'-biphenyl]-3-yl)carbamoyl)pyridin-3-yl)methyl) piperidine-2-carboxylic acid | 701.4 |
| 9 | 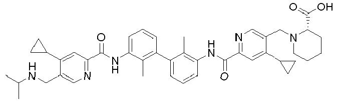 | (*S*)-1-((4-cyclopropyl-6-((3'-(4-cyclo propyl-5-((isopropylamino)methyl)pi colinamido)-2,2'-dimethyl-[1,1'-biphe nyl]-3-yl)carbamoyl)pyridin-3-yl)met hyl)piperidine-2-carboxylic acid | 715.4 |
| 10 | 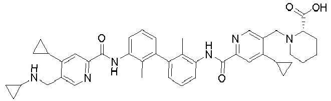 | (*S*)-1-((4-cyclopropyl-6-((3'-(4-cyclo propyl-5-((cyclopropylamino)methyl) picolinamido)-2,2'-dimethyl-[1,1'-bip henyl]-3-yl)carbamoyl)pyridin-3-yl) methyl)piperidine-2-carboxylic acid | 713.4 |
| 11 | 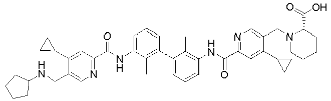 | (*S*)-1-((4-cyclopropyl-6-((3'-(4-cyclo propyl-5-((cyclopentylamino)methyl) picolinamido)-2,2'-dimethyl-[1,1'-bip henyl]-3-yl)carbamoyl)pyridin-3-yl) methyl)piperidine-2-carboxylic acid | 741.7 |
| 12 | 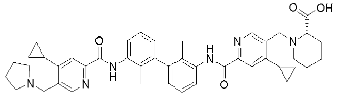 | (*S*)-1-((4-cyclopropyl-6-((3'-(4-cyclo propyl-5-(pyrrolidin-1-ylmethyl)picol inamido)-2,2'-dimethyl-[1,1'-bipheny l]-3-yl)carbamoyl)pyridin-3-yl)methy l)piperidine-2-carboxylic acid | 727.6 |
| 13 | 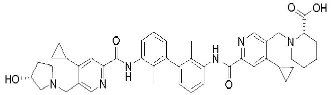 | (*S*)-1-((4-cyclopropyl-6-((3'-(4-cyclo propyl-5-(((*R*)-3-hydroxypyrrolidin-1 -yl)methyl)picolinamido)-2,2'-dimeth yl-[1,1'-biphenyl]-3-yl)carbamoyl)py ridin-3-yl)methyl)piperidine-2-carbo xylic acid | 743.6 |
| 14 | 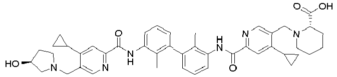 | (*S*)-1-((4-cyclopropyl-6-((3'-(4-cyclo propyl-5-(((*S*)-3-hydroxypyrrolidin-1 -yl)methyl)picolinamido)-2,2'-dimeth yl-[1,1'-biphenyl]-3-yl)carbamoyl)py ridin-3-yl)methyl)piperidine-2-carbo xylic acid | 743.6 |
| 15 | 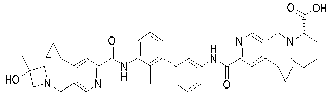 | (*S*)-1-((4-cyclopropyl-6-((3'-(4-cyclo propyl-5-((3-hydroxy-3-methylazetid in-1-yl)methyl)picolinamido)-2,2'-di methyl-[1,1'-biphenyl]-3-yl)carbamo yl)pyridin-3-yl)methyl)piperidine-2-c arboxylic acid | 743.4 |
| 16 | 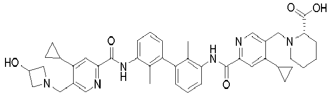 | (*S*)-1-((4-cyclopropyl-6-((3'-(4-cyclo propyl-5-((3-hydroxyazetidin-1-yl)m ethyl)picolinamido)-2,2'-dimethyl-[1, 1'-biphenyl]-3-yl)carbamoyl)pyridin-3-yl)methyl)piperidine-2-carboxylic acid | 729.4 |
| 17 | 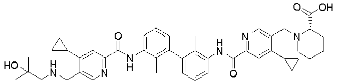 | (*S*)-1-((4-cyclopropyl-6-((3'-(4-cyclo propyl-5-(((2-hydroxy-2-methylprop yl)amino)methyl)picolinamido)-2,2'-dimethyl-[1,1'-biphenyl]-3-yl)carbam oyl)pyridin-3-yl)methyl)piperidine-2-carboxylic acid | 745.4 |
| 18 | 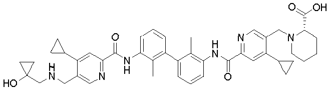 | (*S*)-1-((4-cyclopropyl-6-((3'-(4-cyclo propyl-5-((((1-hydroxycyclopropyl)m ethyl)amino)methyl)picolinamido)-2, 2'-dimethyl-[1,1'-biphenyl]-3-yl)carbamoyl)pyridin-3-yl)methyl)piperidine -2-carboxylic acid | 743.4 |
| 19 | 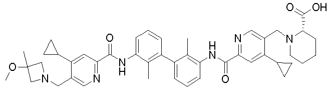 | (*S*)-1-((4-cyclopropyl-6-((3'-(4-cyclo propyl-5-((3-methoxy-3-methylazetid in-1-yl)methyl)picolinamido)-2,2'-di methyl-[1,1'-biphenyl]-3-yl)carbamo yl)pyridin-3-yl)methyl)piperidine-2-c arboxylic acid | 757.4 |
| 20 | 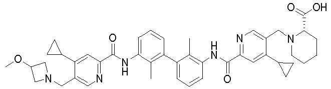 | (*S*)-1-((4-cyclopropyl-6-((3'-(4-cyclo propyl-5-((3-methoxyazetidin-1-yl)m ethyl)picolinamido)-2,2'-dimethyl-[1, 1'-biphenyl]-3-yl)carbamoyl)pyridin-3-yl)methyl)piperidine-2-carboxylic acid | 743.4 |
| 21 | 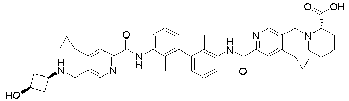 | (*S*)-1-((4-cyclopropyl-6-((3'-(4-cyclo propyl-5-((((1*s*,3*s*)-3-hydroxycyclobu tyl)amino)methyl)picolinamido)-2,2'-dimethyl-[1,1'-biphenyl]-3-yl)carbam oyl)pyridin-3-yl)methyl)piperidine-2-carboxylic acid | 743.4 |
| 22 | 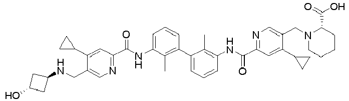 | (*S*)-1-((4-cyclopropyl-6-((3'-(4-cyclo propyl-5-((((1*r*,3*r*)-3-hydroxycyclobu tyl)amino)methyl)picolinamido)-2,2'-dimethyl-[1,1'-biphenyl]-3-yl)carbam oyl)pyridin-3-yl)methyl)piperidine-2-carboxylic acid | 743.4 |
| 23 | 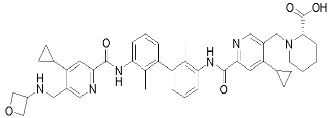 | (*S*)-1-((4-cyclopropyl-6-((3'-(4-cyclo propyl-5-((oxetan-3-ylamino)methyl) picolinamido)-2,2'-dimethyl-[1,1'-bip henyl]-3-yl)carbamoyl)pyridin-3-yl) methyl)piperidine-2-carboxylic acid | 729.4 |
| 24 | 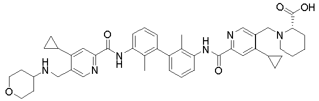 | (*S*)-1-((4-cyclopropyl-6-((3'-(4-cyclo propyl-5-(((tetrahydro-2*H*-pyran-4-y l)amino)methyl)picolinamido)-2,2'-di methyl-[1,1'-biphenyl]-3-yl)carbamo yl)pyridin-3-yl)methyl)piperidine-2-c arboxylic acid | 757.4 |
| 25 | 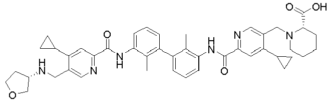 | (*S*)-1-((4-cyclopropyl-6-((3'-(4-cyclo propyl-5-((((*S*)-tetrahydrofuran-3-yl) amino)methyl)picolinamido)-2,2'-di methyl-[1,1'-biphenyl]-3-yl)carbamo yl)pyridin-3-yl)methyl)piperidine-2-c arboxylic acid | 743.6 |
| 26 | 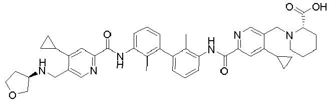 | (*S*)-1-((4-cyclopropyl-6-((3'-(4-cyclo propyl-5-((((*R*)-tetrahydrofuran-3-yl) amino)methyl)picolinamido)-2,2'-di methyl-[1,1'-biphenyl]-3-yl)carbamo yl)pyridin-3-yl)methyl)piperidine-2-c arboxylic acid | 743.4 |
| 27 | 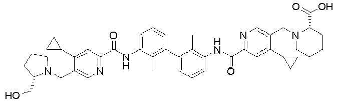 | (*S*)-1-((4-cyclopropyl-6-((3'-(4-cyclo propyl-5-(((*S*)-2-(hydroxymethyl)pyr rolidin-1-yl)methyl)picolinamido)-2, 2'-dimethyl-[1,1'-biphenyl]-3-yl)carb amoyl)pyridin-3-yl)methyl)piperidine -2-carboxylic acid | 757.4 |
| 28 | 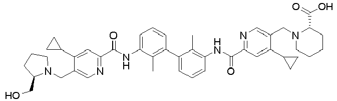 | (*S*)-1-((4-cyclopropyl-6-((3'-(4-cyclo propyl-5-(((*R*)-2-(hydroxymethyl)pyr rolidin-1-yl)methyl)picolinamido)-2, 2'-dimethyl-[1,1'-biphenyl]-3-yl)carb amoyl)pyridin-3-yl)methyl)piperidine -2-carboxylic acid | 757.4 |

### Example 29: Preparation of (S)-1-((4-cyclopropyl-6-((3'-(4-cyclopropyl-5-((((R)-1-ethoxy-3-hydroxy-1-oxo propan-2-yl)amino)methyl)picolinamido)-2,2'-dimethyl-[1,1'-biphenyl]-3-yl)c arbamoyl)pyridin-3-yl)methyl)piperidine-2-carboxylic acid

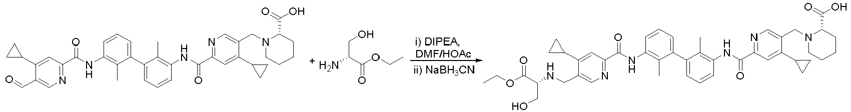

(*S*)-1-((4-cyclopropyl-6-((3'-(4-cyclopropyl-5-formylpicolinamido)-2,2'-dimethyl-[1,1'-biphenyl]-3-yl)carbamoyl)pyridin-3-yl)methyl)piperidine-2-carboxylic acid (50.0 mg, 0.074 mmol), d-serine ethyl ester hydrochloride (63.0 mg, 0.372 mmol) and DIPEA (0.07 mL, 0.372 mmol) were dissolved in DMF/HOAc (3 mL/1 mL), and the reaction solution was stirred at room temperature for 2 hrs. The reaction solution was added with NaBH₃CN (23.0 mg, 0.372 mmol), and stirred at room temperature for 16 hrs. After the reaction was completed, 3 drops of methanol was added to terminate the reaction. The crude product was separated by reversed-phase column chromatography to obtain (*S*)-1-((4-cyclopropyl-6-((3'-(4-cyclopropyl-5-((((*R*)-1-ethoxy-3-hydroxy-1-oxopropan-2-yl)am ino)methyl)picolinamido)-2,2'-dimethyl-[1,1'-biphenyl]-3-yl)carbamoyl)pyridin-3-yl)methyl) piperidine-2-carboxylic acid (32 mg, yield: 55 %). MS m/z (ESI): 789.4 [M+H]⁺.

¹H NMR (400 MHz, DMSO-*d*₆) δ 10.36 (s, 1H), 10.33 (s, 1H), 8.71 (s, 1H), 8.57 (s, 1H), 7.82 (dd, *J* = 15.3, 8.0 Hz, 2H), 7.64 (s, 1H), 7.60 (s, 1H), 7.33 (t, *J* = 7.9 Hz, 2H), 7.00 (t, *J* = 6.9 Hz, 2H), 4.45 (brs, 2H), 4.22 (q, *J* = 7.1, 6.6 Hz, 2H), 3.92 (brs, 2H), 3.00 - 2.85 (m, 2H), 2.45 - 2.40 (m, 2H), 2.36 - 2.28 (m, 2H), 2.00 (d, *J* = 3.3 Hz, 6H), 1.80 - 1.70 (m, 2H), 1.65 - 1.40 (m, 4H), 1.25 (t, *J* = 7.1 Hz, 3H), 1.21 - 1.08 (m, 4H), 1.00 - 0.75 (m, 4H).

### Example 30: Preparation of (S)-1-((6-((3'-(5-((((R)-1-carboxy-2-hydroxyethyl)amino)methyl)-4-cyclopropylpi colinamido)-2,2'-dimethyl-[1,1'-biphenyl]-3-yl)carbamoyl)-4-cyclopropylpy ridin-3-yl)methyl)piperidine-2-carboxylic acid

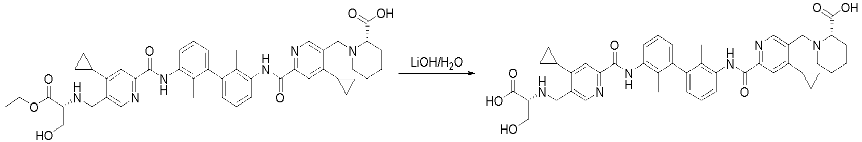

(*S*)-1-((4-cyclopropyl-6-((3'-(4-cyclopropyl-5-((((*R*)-1-ethoxy-3-hydroxy-1-oxopropan-2-yl)amino)methyl)picolinamido)-2,2'-dimethyl-[1,1'-biphenyl]-3-yl)carbamoyl)pyridin-3-yl )methyl)piperidine-2-carboxylic acid (17.0 mg, 0.022 mmol) was dissolved in methanol/tetrahydrofuran/ water (1 mL/1 mL/1 mL), and then the reaction solution was added with LiOH·H₂O (9 mg, 0.22 mmol) and reacted at room temperature for 1 hr. After the reaction was completed, the reaction solution was concentrated and adjusted to weak acidity with HCl (1 N), and the crude product was separated by reversed-phase column chromatography to obtain (*S*)-1-((6-((3'-(5-((((*R*)-1-carboxy-2-hydroxyethyl)amino)methyl)-4-cyclopropylpicolinamido)-2 ,2'-dimethyl-[1,1'-biphenyl]-3-yl)carbamoyl)-4-cyclopropylpyridin-3-yl)methyl)piperidine-2-carboxylic acid (12 mg, yield: 73%). MS m/z (ESI): 761.4 [M+H]⁺.

¹H NMR (400 MHz, DMSO-*d*₆) δ 10.32 (s, 1H), 10.30 (s, 1H), 8.59 (s, 1H), 8.52 (s, 1H), 7.87 (dd, *J* = 8.0, 4.2 Hz, 2H), 7.60 (s, 1H), 7.59 (s, 1H), 7.32 (t, *J* = 7.8 Hz, 2H), 6.98 (d, *J* = 7.7 Hz, 2H), 4.16 (d, *J* = 13.8 Hz, 1H), 4.02 (dd, *J* = 13.7, 5.9 Hz, 2H), 3.76 - 3.63 (m, 2H), 3.24 - 3.16 (m, 2H), 2.90 - 2.79 (m, 1H), 2.48 - 2.40 (m, 1H), 2.37 - 2.20 (m, 3H), 2.01 (s, 6H), 1.83 - 1.71 (m, 2H), 1.53 - 1.38 (m, 4H), 1.18 - 1.05 (m, 4H), 0.94 - 0.73 (m, 4H).

Examples 31 to 34 were prepared by selecting corresponding raw materials according to all or part of the synthesis method in Example 29 or 30.

| Example No. | Structure | Name | MS: *m*/*z* [M+1]⁺ |
|---|---|---|---|
| 31 | 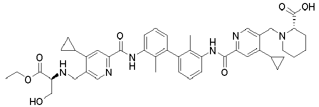 | (*S*)-1-((4-cyclopropyl-6-((3'-(4-cyclopr opyl-5-((((*S*)-1-ethoxy-3-hydroxy-1-ox opropan-2-yl)amino)methyl)picolinam ido)-2,2'-dimethyl-[1,1'-biphenyl]-3-y l)carbamoyl)pyridin-3-yl)methyl)piper idine-2-carboxylic acid | 789.4 |
| 32 | 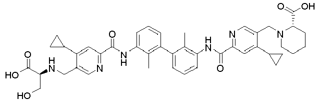 | (*S*)-1-((6-((3'-(5-((((*S*)-1-carboxy-2-hy droxyethyl)amino)methyl)-4-cyclopro pylpicolinamido)-2,2'-dimethyl-[1,1'-b iphenyl]-3-yl)carbamoyl)-4-cycloprop ylpyridin-3-yl)methyl)piperidine-2-car boxylic acid | 761.4 |
| 33 | 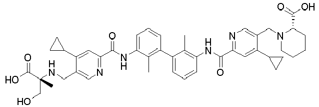 | (*S*)-1-((6-((3'-(5-((((*R*)-2-carboxy-1-hy droxypropan-2-yl)amino)methyl)-4-cy clopropylpicolinamido)-2,2'-dimethyl-[1,1'-biphenyl]-3-yl)carbamoyl)-4-cycl opropylpyridin-3-yl)methyl)piperidine -2-carboxylic acid | 775.4 |
| 34 | 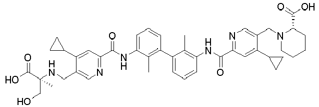 | (*S*)-1-((6-((3'-(5-((((*S*)-2-carboxy-1-hy droxypropan-2-yl)amino)methyl)-4-cy clopropylpicolinamido)-2,2'-dimethyl-[1,1'-biphenyl]-3 -yl)carbamoyl)-4-cycl opropylpyridin-3-yl)methyl)piperidine -2-carboxylic acid | 775.4 |

### Example 35: Preparation of ((4-cyclopropyl-6-((3'-(4-cyclopropyl-5-(((2-hydroxyethyl)amin o)methyl)picolinamido)-2,2'-dimethyl-[1,1'-biphenyl]-3-yl)carbamoyl)pyridi n-3-yl)methyl)-D-serine

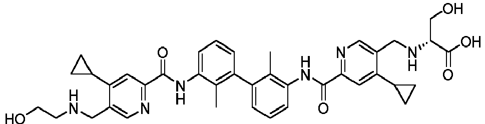

### Step 1: Synthesis of ethyl ((4-cyclopropyl-6-((3'-(4-cyclopropyl-5-(dimethoxymethyl)pic olinamido)-2,2'-dimethyl-[1,1'-biphenyl]-3-yl)carbamoyl)pyridin-3-yl)methyl)-D-serinate

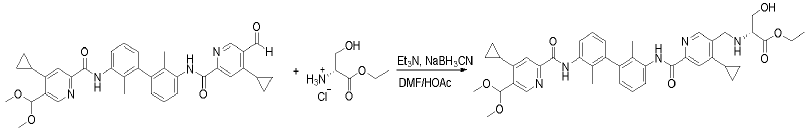

4-cyclopropyl-N-(3'-(4-cyclopropyl-5-(dimethoxymethyl)picolinamido)-2,2'-dimethyl-[1,1'-b iphenyl]-3-yl)-5-formylpicolinamide (300 mg, 0.497 mmol) was dissolved in DMF (3 mL), and the reaction solution was added with d-serine ethyl ester hydrochloride (420 mg, 2.48 mmol), triethylamine (250 mg, 2.48 mmol) and acetic acid (1 mL). The reaction solution was stirred at room temperature for 2 hrs, then added with NaBH₃CN (94 mg, 1.49 mmol) and stirred at room temperature overnight. After the reaction was completed as detected by LCMS, the reaction solution was directly separated by reversed-phase column chromatography to obtain ethyl ((4-cyclopropyl-6-((3'-(4-cyclopropyl-5-(dimethoxymethyl)picolinamido)-2,2'-dimethyl-[1,1'-biphe nyl]-3-yl)carbamoyl)pyridin-3-yl)methyl)-D-serinate (300 mg, yield: 84%). MS m/z (ESI): 722.4 [M+H]⁺.

### Step 2: Synthesis of ethyl ((4-cyclopropyl-6-((3'-(4-cyclopropyl-5-formylpicolinamido)-2,2'-dimethyl-[1,1'-biphenyl]-3-yl)carbamoyl)pyridin-3-yl)methyl)-D-serinate

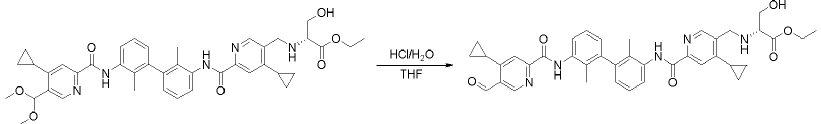

Ethyl ((4-cyclopropyl-6-((3'-(4-cyclopropyl-5-(dimethoxymethyl)picolinamido)-2,2'-dimet hyl-[1,1'-biphenyl]-3-yl)carbamoyl)pyridin-3-yl)methyl)-D-serinate (300 mg, 0.416 mmol) w as dissolved in THF (10 mL), and the reaction solution was added with a hydrochloric acid solution (10 mL, 4 M) with stirring and stirred at room temperature for 1 hr. After the reaction was completed as detected by LCMS, the reaction system was cooled in a n ice bath and neutralized by adding a saturated aqueous NaHCO₃ solution. The reaction solution was extracted 3 times with ethyl acetate (20 mL), the organic phases were co mbined, washed with brine, dried over anhydrous Na₂SO₄, filtered and concentrated to o btain a crude product (260 mg, yield: 93%), which was directly used in the next step. MS m/z (ESI): 676.6 [M+H]⁺.

### Step 3: Synthesis of ethyl ((4-cyclopropyl-6-((3'-(4-cyclopropyl-5-(((2-hydroxyethyl)ami no)methyl)picolinamido)-2,2'-dimethyl-[1,1'-biphenyl]-3-yl)carbamoyl)pyridin-3-yl)meth yl)-D-serinate (Example 35-1)

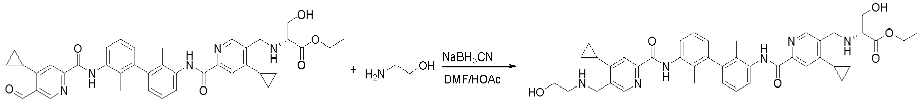

Ethyl ((4-cyclopropyl-6-((3'-(4-cyclopropyl-5-formylpicolinamido)-2,2'-dimethyl-[1,1'-bip henyl]-3-yl)carbamoyl)pyridin-3-yl)methyl)-D-serinate (60 mg, 0.089 mmol) was dissolved in DMF (3 mL), and the reaction solution was added with 2-aminoethanol (54 mg, 0.8 9 mmol) and acetic acid (1 mL). The reaction solution was stirred at room temperature for 2 hrs, then added with NaBH₃CN (17 mg, 0.267 mmol) and stirred overnight. After the reaction was completed as detected by LCMS, the reaction solution was directly se parated by reversed-phase column chromatography to obtain ethyl ((4-cyclopropyl-6-((3'-(4 -cyclopropyl-5-(((2-hydroxyethyl)amino)methyl)picolinamido)-2,2'-dimethyl-[1,1'-biphenyl]-3-yl)carbamoyl)pyridin-3-yl)methyl)-D-serinate (45 mg, yield: 70%). MS m/z (ESI): 721.4 [M+H]⁺; 361.2 [1/2M+H]⁺.

¹H NMR (400 MHz, DMSO-*d*₆) δ 10.31 (s, 2H), 8.59 (s, 1H), 8.54 (s, 1H), 7.87 (d, *J* = 8.2 Hz, 2H), 7.60 (s, 1H), 7.59 (s, 1H), 7.32 (t, *J* = 7.8 Hz, 2H), 6.98 (d, *J* = 7.6 Hz, 2H), 4.85 (t, *J* = 5.8 Hz, 1H), 4.15 - 4.00 (m, 5H), 3.90 (d, *J* = 13.7 Hz, 1H), 3.61 (t, *J* = 5.2 Hz, 2H), 3.56 - 3.48 (m, 2H), 2.74 (brs, 2H), 2.37 - 2.22 (m, 2H), 2.00 (s, 6H), 1.20 (t, *J* = 7.1 Hz, 3H), 1.19 - 1.06 (m, 4H), 0.92 - 0.79 (m, 4H).

### Step 4: Synthesis of ((4-cyclopropyl-6-((3'-(4-cyclopropyl-5-(((2-hydroxyethyl)amino)met hyl)picolinamido)-2,2'-dimethyl-[1,1'-biphenyl]-3-yl)carbamoyl)pyridin-3-yl)methyl)-D-seri ne

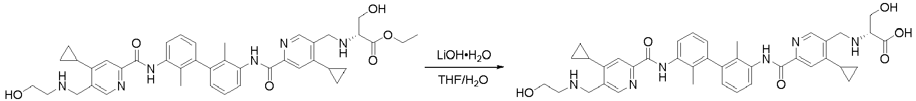

Ethyl ((4-cyclopropyl-6-((3'-(4-cyclopropyl-5-(((2-hydroxyethyl)amino)methyl)picolinamido)-2,2'-dimet hyl-[1,1'-biphenyl]-3-yl)carbamoyl)pyridin-3-yl)methyl)-D-serinate (20 mg, 0.028 mmol) was dissolved in THF (3 mL) and water (3 mL), and then the reaction solution was added with LiOH·H₂O (6 mg, 0.14 mmol) and stirred at room temperature for 1 hr. After the reaction was completed as detected by LCMS, the reaction solution was adjusted to pH < 7 with acetic acid, and directly separated by reversed-phase column chromatography to obtain ((4-cyclopropyl-6-((3'-(4-cyclopropyl-5-(((2-hydroxyethyl)amino)methyl)picolinamido)-2,2'-di methyl-[1,1'-biphenyl]-3-yl)carbamoyl)pyridin-3-yl)methyl)-D-serine (15 mg, yield: 78%). MS m/z (ESI): 693.4 [M+H]⁺; 347.2 [1/2M+H]⁺.

¹H NMR (400 MHz, DMSO-*d*₆) δ 10.32 (s, 2H), 8.61 (s, 1H), 8.59 (s, 1H), 7.87 (d, *J* = 8.1 Hz, 2H), 7.61 (s, 1H), 7.60 (s, 1H), 7.32 (t, *J* = 7.8 Hz, 2H), 6.98 (d, *J* = 7.7 Hz, 1H), 4.20 - 4.10 (m, 3H), 3.99 (d, *J* = 13.7 Hz, 1H), 3.65 (t, *J* = 5.7 Hz, 2H), 3.57 (t, *J* = 5.6 Hz, 2H), 3.28 (t, *J* = 5.0 Hz, 1H), 2.80 (t, *J* = 5.6 Hz, 2H), 2.40 - 2.22 (m, 2H), 2.00 (s, 6H), 1.20 - 1.08 (m, 4H), 0.95 - 0.80 (m, 4H).

### Example 36: Preparation of ((4-cyclopropyl-6-((3'-(4-cyclopropyl-5-((3-hydroxy-3-methy lazetidin-1-yl)methyl)picolinamido)-2,2'-dimethyl-[1,1'-biphenyl]-3-yl)carbam oyl)pyridin-3-yl)methyl)-D-serine

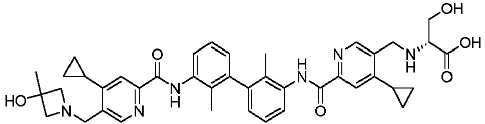

### Step 1: Synthesis of ethyl ((4-cyclopropyl-6-((3'-(4-cyclopropyl-5-((3-hydroxy-3-methyla zetidin-1-yl)methyl)picolinamido)-2,2'-dimethyl-[1,1'-biphenyl]-3-yl)carbamoyl)pyridin-3 -yl)methyl)-D-serinate (Example 36-1)

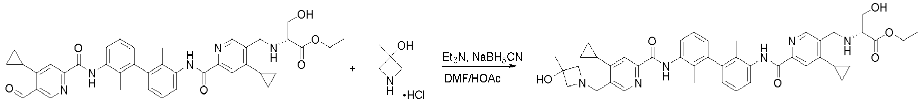

Ethyl ((4-cyclopropyl-6-((3'-(4-cyclopropyl-5-formylpicolinamido)-2,2'-dimethyl-[1,1'-biphenyl]-3-yl )carbamoyl)pyridin-3-yl)methyl)-D-serinate (60 mg, 0.089 mmol) was dissolved in DMF (3 mL), and 3-methylazetidine-3-ol hydrochloride (33 mg, 0.267 mmol), triethylamine (27 mg, 0.267 mmol) and acetic acid (1 mL) were successively added. The reaction solution was stirred at room temperature for 2 hrs, then added with NaBH₃CN (17 mg, 0.267 mmol) and stirred overnight. After the reaction was completed as detected by LCMS, the reaction solution was directly separated by reversed-phase column chromatography to obtain ethyl ((4-cyclopropyl-6-((3'-(4-cyclopropyl-5-((3-hydroxy-3-methylazetidin-1-yl)methyl)picolinamid o)-2,2'-dimethyl-[1,1'-biphenyl]-3-yl)carbamoyl)pyridin-3-yl)methyl)-D-serinate (40 mg, yield: 60%). MS m/z (ESI): 747.4 [M+H]⁺; 374.2 [1/2M+H]⁺.

¹H NMR (400 MHz, DMSO-*d*₆) δ 10.31 (s, 1H), 10.29 (s, 1H), 8.54 (s, 1H), 8.48 (s, 1H), 7.88 (d, *J* = 8.1 Hz, 2H), 7.59 (s, 2H), 7.31 (t, *J* = 7.8 Hz, 2H), 6.98 (d, *J* = 7.5 Hz, 2H), 5.19 (s, 1H), 4.85 (s, 1H), 4.15 - 4.01 (m, 3H), 3.90 (dd, *J* = 13.5, 5.7 Hz, 1H), 3.85 (s, 2H), 3.61 (brs, 2H), 3.23 (d, *J* = 6.8 Hz, 2H), 2.99 (d, *J* = 6.4 Hz, 2H), 2.35 - 2.19 (m, 2H), 2.00 (s, 6H), 1.38 (s, 3H), 1.20 (t, *J* = 7.1 Hz, 3H), 1.12 (d, *J* = 8.1 Hz, 4H), 0.90 - 0.80 (m, 4H).

### Step 2: Synthesis of ((4-cyclopropyl-6-((3'-(4-cyclopropyl-5-((3-hydroxy-3-methylazetidin-1-yl)methyl)picolinam ido)-2,2'-dimethyl-[1,1'-biphenyl]-3-yl)carbamoyl)pyridin-3-yl)methyl)-D-serine

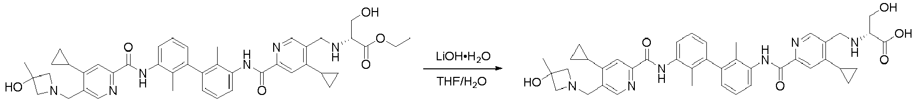

Ethyl ((4-cyclopropyl-6-((3'-(4-cyclopropyl-5-((3-hydroxy-3-methylazetidin-1-yl)methyl)p icolinamido)-2,2'-dimethyl-[1,1'-biphenyl]-3-yl)carbamoyl)pyridin-3-yl)methyl)-D-serinate (15 mg, 0.02 mmol) was dissolved in THF (3 mL) and water (3 mL), and then the reactio n solution was added with LiOH·H₂O (4 mg, 0.10 mmol) and stirred at room temperatu re for 1 hr. After the reaction was completed as detected by LCMS, the reaction solutio n was adjusted to pH < 7 with acetic acid and then separated by reversed-phase column chromatography to obtain ((4-cyclopropyl-6-((3'-(4-cyclopropyl-5-((3-hydroxy-3-methylazeti din-1-yl)methyl)picolinamido)-2,2'-dimethyl-[1,1'-biphenyl]-3-yl)carbamoyl)pyridin-3-yl)methy l)-D-serine (10 mg, yield: 69%). MS m/z (ESI): 719.4 [M+H]⁺; 360.2 [1/2M+H]⁺.

¹H NMR (400 MHz, DMSO-*d*₆) δ 10.33 (s, 1H), 10.30 (s, 1H), 8.59 (s, 1H), 8.48 (s, 1H), 7.88 (d, *J* = 8.0 Hz, 1H), 7.87 (d, *J* = 8.0 Hz, 1H), 7.60 (s, 1H), 7.59 (s, 1H), 7.32 (t, *J* = 7.8 Hz, 2H), 6.98 (d, *J* = 7.6 Hz, 2H), 5.21 (s, 1H), 4.13 (d, *J* = 13.8 Hz, 1H), 3.98 (d, *J* = 13.5 Hz, 1H), 3.86 (s, 2H), 3.60 (d, *J* = 5.4 Hz, 2H), 3.24 (d, *J* = 6.8 Hz, 2H), 3.25 - 3.15 (m, 1H), 3.00 (d, *J* = 6.7 Hz, 2H), 2.36 - 2.24 (m, 2H), 2.01 (s, 6H), 1.38 (s, 3H), 1.19 - 1.08 (m, 4H), 0.90 - 0.82 (m, 4H).

Examples 37 to 66 were prepared by selecting corresponding raw materials according to all or part of the synthesis method in Example 35 or 36.

| Example No. | Structure | Name | MS: *m*/*z* [M+1]⁺ |
|---|---|---|---|
| 37-1 | 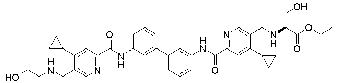 | Ethyl ((4-cyclopropyl-6-((3'-(4-cycl opropyl-5-(((2-hydroxyethyl)amino) methyl)picolinamido)-2,2'-dimethyl-[1,1'-biphenyl]-3-yl)carbamoyl)pyrid in-3-yl)methyl)-L-serinate | 721.5 |
| 37 | 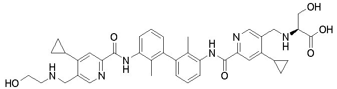 | ((4-cyclopropyl-6-((3'-(4-cyclopropyl -5-(((2-hydroxyethyl)amino)methyl) picolinamido)-2,2'-dimethyl-[1,1'-bip henyl]-3-yl)carbamoyl)pyridin-3-yl) methyl)-L-serine | 693.6 |
| 38-1 | 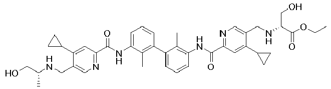 | Ethyl ((4-cyclopropyl-6-((3'-(4-cycl opropyl-5-((((*R*)-1-hydroxypropan-2-yl)amino)methyl)picolinamido)-2,2'-dimethyl-[1,1'-biphenyl]-3-yl)carbam oyl)pyridin-3-yl)methyl)-D-serinate | 735.6 |
| 38 | 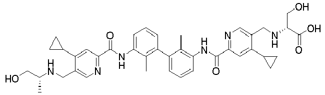 | ((4-cyclopropyl-6-((3'-(4-cyclopropyl -5-((((*R*)-1-hydroxypropan-2-yl)amin o)methyl)picolinamido)-2,2'-dimethy l-[1,1'-biphenyl]-3-yl)carbamoyl)pyri din-3-yl)methyl)-D-serine | 707.4 |
| 39-1 | 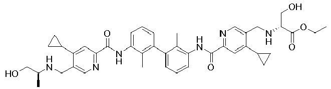 | Ethyl ((4-cyclopropyl-6-((3'-(4-cycl opropyl-5-((((*S*)-1-hydroxypropan-2-yl)amino)methyl)picolinamido)-2,2'-dimethyl-[1,1'-biphenyl]-3-yl)carbam oyl)pyridin-3-yl)methyl)-D-serinate | 735.6 |
| 39 | 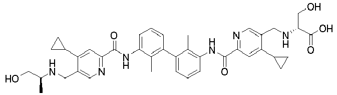 | ((4-cyclopropyl-6-((3'-(4-cyclopropyl -5-((((*S*)-1-hydroxypropan-2-yl)amin o)methyl)picolinamido)-2,2'-dimethy l-[1,1'-biphenyl]-3-yl)carbamoyl)pyri din-3-yl)methyl)-D-serine | 606.4 |
| 40 | 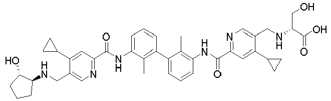 | ((4-cyclopropyl-6-((3'-(4-cyclopropyl -5-((((1*S*,2*S*)-2-hydroxycyclopentyl)a mino)methyl)picolinamido)-2,2'-dim ethyl-[1,1'-biphenyl]-3-yl)carbamoy l)pyridin-3-yl)methyl)-D-serine | 733.4 |
| 41 | 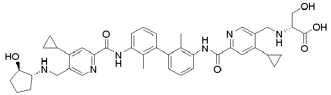 | ((4-cyclopropyl-6-((3'-(4-cyclopropyl -5-((((1*R*,2*R*)-2-hydroxycyclopentyl) amino)methyl)picolinamido)-2,2'-di methyl-[1,1'-biphenyl]-3-yl)carbamoyl)pyridin-3-yl)methyl)-D-serine | 733.4 |
| 42 | 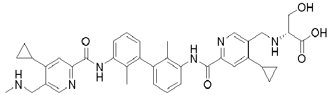 | ((4-cyclopropyl-6-((3'-(4-cyclopropyl -5-((methylamino)methyl)picolinami do)-2,2'-dimethyl-[1,1'-biphenyl]-3-y l)carbamoyl)pyridin-3-yl)methyl)-D-serine | 663.4 |
| 43 | 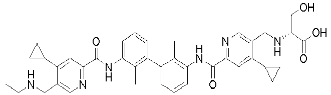 | ((4-cyclopropyl-6-((3'-(4-cyclopropyl -5-((ethylamino)methyl)picolinamid o)-2,2'-dimethyl-[1,1'-biphenyl]-3-yl) carbamoyl)pyridin-3-yl)methyl)-D-se rine | 677.4 |
| 44 | 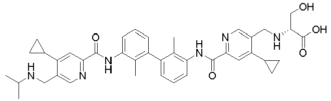 | ((4-cyclopropyl-6-((3'-(4-cyclopropyl -5-((isopropylamino)methyl)picolina mido)-2,2'-dimethyl-[1,1'-biphenyl]-3-yl)carbamoyl)pyridin-3-yl)methyl) -D-serine | 691.4 |
| 45 | 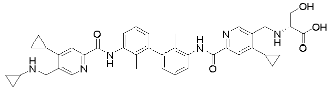 | ((4-cyclopropyl-6-((3'-(4-cyclopropyl -5-((cyclopropylamino)methyl)picoli namido)-2,2'-dimethyl-[1,1'-bipheny l]-3-yl)carbamoyl)pyridin-3-yl)meth yl)-D-serine | 689.4 |
| 46 | 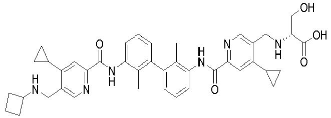 | ((4-cyclopropyl-6-((3'-(4-cyclopropyl -5-((cyclobutylamino)methyl)picolin amido)-2,2'-dimethyl-[1,1'-biphenyl]-3-yl)carbamoyl)pyridin-3-yl)methyl) -D-serine | 703.4 |
| 47 | 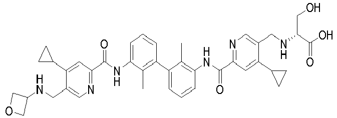 | ((4-cyclopropyl-6-((3'-(4-cyclopropyl -5-((oxetan-3-ylamino)methyl)picoli namido)-2,2'-dimethyl-[1,1'-bipheny l]-3-yl)carbamoyl)pyridin-3-yl)meth yl)-D-serine | 705.4 |
| 48 | 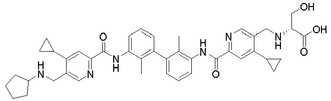 | ((4-cyclopropyl-6-((3'-(4-cyclopropyl -5-((cyclopentylamino)methyl)picoli namido)-2,2'-dimethyl-[1,1'-bipheny l]-3-yl)carbamoyl)pyridin-3-yl)meth yl)-D-serine | 717.4 |
| 49 | 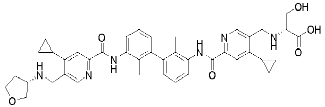 | ((4-cyclopropyl-6-((3'-(4-cyclopropyl -5-((((*S*)-tetrahydrofuran-3-yl)amino) methyl)picolinamido)-2,2'-dimethyl-[1,1'-biphenyl]-3-yl)carbamoyl)pyrid in-3-yl)methyl)-D-serine | 719.4 |
| 50 | 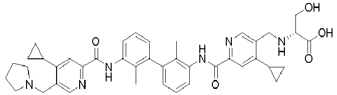 | ((4-cyclopropyl-6-((3'-(4-cyclopropyl -5-(pyrrolidin-1-ylmethyl)picolinami do)-2,2'-dimethyl-[1,1'-biphenyl]-3-y l)carbamoyl)pyridin-3-yl)methyl)-D-serine | 703.4 |
| 51 | 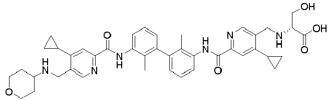 | ((4-cyclopropyl-6-((3'-(4-cyclopropyl -5-(((tetrahydro-2*H*-pyran-4-yl)amin o)methyl)picolinamido)-2,2'-dimethy 1-[1,1'-biphenyl]-3-yl)carbamoyl)pyri din-3-yl)methyl)-D-serine | 733.4 |
| 52 | 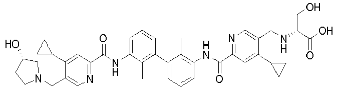 | ((4-cyclopropyl-6-((3'-(4-cyclopropyl -5-(((*S*)-3-hydroxypyrrolidin-1-yl)me thyl)picolinamido)-2,2'-dimethyl-[1,1 '-biphenyl]-3-yl)carbamoyl)pyridin-3 -yl)methyl)-D-serine | 719.4 |
| 53-1 | 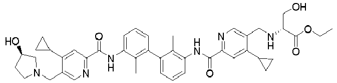 | Ethyl ((4-cyclopropyl-6-((3'-(4-cycl opropyl-5-(((*R*)-3-hydroxypyrrolidin-1-yl)methyl)picolinamido)-2,2'-dimet hyl-[1,1'-biphenyl]-3-yl)carbamoyl)p yridin-3-yl)methyl)-D-serinate | 747.6 |
| 53 | 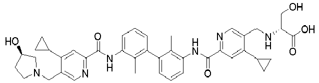 | ((4-cyclopropyl-6-((3'-(4-cyclopropyl -5-(((*R*)-3-hydroxypyrrolidin-1-yl)m ethyl)picolinamido)-2,2'-dimethyl-[1, 1'-biphenyl]-3-yl)carbamoyl)pyridin-3 -yl)methyl)-D-serine | 719.6 |
| 54 | 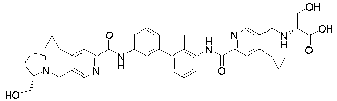 | ((4-cyclopropyl-6-((3'-(4-cyclopropyl -5-(((*S*)-2-(hydroxymethyl)pyrrolidin -1-yl)methyl)picolinamido)-2,2'-dim ethyl-[1,1'-biphenyl]-3-yl)carbamoy l)pyridin-3-yl)methyl)-D-serine | 733.4 |
| 55 | 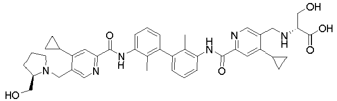 | ((4-cyclopropyl-6-((3'-(4-cyclopropyl -5-(((*R*)-2-(hydroxymethyl)pyrrolidin -1-yl)methyl)picolinamido)-2,2'-dim ethyl-[1,1'-biphenyl]-3-yl)carbamoy l)pyridin-3-yl)methyl)-D-serine | 733.4 |
| 56 | 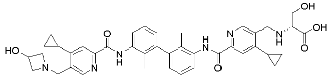 | ((4-cyclopropyl-6-((3'-(4-cyclopropyl -5-((3-hydroxyazetidin-1-yl)methyl)p icolinamido)-2,2'-dimethyl-[1,1'-biph enyl]-3-yl)carbamoyl)pyridin-3-yl)m ethyl)-D-serine | 705.4 |
| 57 | 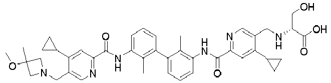 | ((4-cyclopropyl-6-((3'-(4-cyclopropyl -5-((3-methoxy-3-methylazetidin-1-y 1)methyl)picolinamido)-2,2'-dimethyl -[1,1'-biphenyl]-3-yl)carbamoyl)pyri din-3-yl)methyl)-D-serine | 733.4 |
| 58 | 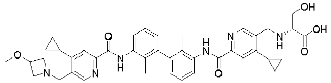 | ((4-cyclopropyl-6-((3'-(4-cyclopropyl -5-((3-methoxyazetidin-1-yl)methyl) picolinamido)-2,2'-dimethyl-[1,1'-bip henyl]-3-yl)carbamoyl)pyridin-3-yl) methyl)-D-serine | 719.4 |
| 59 | 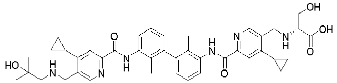 | ((4-cyclopropyl-6-((3'-(4-cyclopropyl -5-(((2-hydroxy-2-methylpropyl)ami no)methyl)picolinamido)-2,2'-dimethyl-[1,1'-biphenyl]-3-yl)carbamoyl)py ridin-3-yl)methyl)-D-serine | 721.4 |
| 60 | 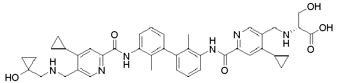 | ((4-cyclopropyl-6-((3'-(4-cyclopropyl -5-((((1-hydroxycyclopropyl)methyl) amino)methyl)picolinamido)-2,2'-di methyl-[1,1'-biphenyl]-3-yl)carbamo yl)pyridin-3-yl)methyl)-D-serine | 719.4 |
| 61 | 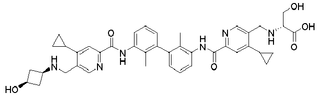 | ((4-cyclopropyl-6-((3'-(4-cyclopropyl -5-((((1*s*,3*s*)-3-hydroxycyclobutyl)a mino)methyl)picolinamido)-2,2'-dim ethyl-[1,1'-biphenyl]-3-yl)carbamoy l)pyridin-3-yl)methyl)-D-serine | 719.4 |
| 62 | 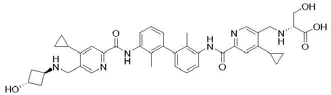 | ((4-cyclopropyl-6-((3'-(4-cyclopropyl -5-((((1*r*,3*r*)-3-hydroxycyclobutyl)a mino)methyl)picolinamido)-2,2'-dim ethyl-[1,1'-biphenyl]-3-yl)carbamoy l)pyridin-3-yl)methyl)-D-serine | 719.4 |
| 63 | 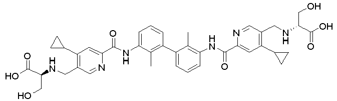 | ((6-((3'-(5-((((*R*)-1-carboxy-2-hydrox yethyl)amino)methyl)-4-cyclopropyl picolinamido)-2,2'-dimethyl-[1,1'-bip henyl]-3-yl)carbamoyl)-4-cyclopropy lpyridin-3-yl)methyl)-L-serine | 737.4 |
| 64 | 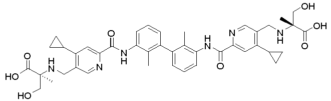 | (*S*)-2-(((6-((3'-(5-((((*R*)-2-carboxy-1-hydroxypropan-2-yl)amino)methyl)-4-cyclopropylpicolinamido)-2,2'-dim ethyl-[1,1'-biphenyl]-3-yl)carbamoy l)-4-cyclopropylpyridin-3-yl)methyl) amino)-3-hydroxy-2-methylpropanoc i acid | 765.4 |
| 65 | 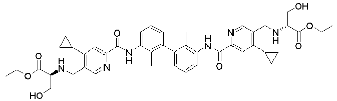 | Ethyl ((4-cyclopropyl-6-((3'-(4-cycl opropyl-5-((((*R*)-1-ethoxy-3-hydroxy -1-oxopropan-2-yl)amino)methyl)pic olinamido)-2,2'-dimethyl-[1,1'-biphe nyl]-3-yl)carbamoyl)pyridin-3-yl)me thyl)-L-serinate | 793.4 |
| 66 | 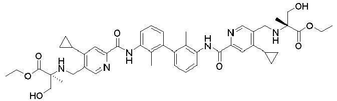 | Ethyl (*S*)-2-(((4-cyclopropyl-6-((3'-(4-cyclopropyl-5-((((*R*)-1-ethoxy-3-h ydroxy-2-methyl-1-oxopropan-2-yl)a mino)methyl)picolinamido)-2,2'-dim ethyl-[1,1'-biphenyl]-3-yl)carbamoy l)pyridin-3-yl)methyl)amino)-3-hydr oxy-2-methylpropanoate | 821.4 |

### Example 67: Preparation of (2S,2'S)-2,2'-((((((2,2'-dimethyl-[1,1'-biphenyl]-3,3'-diyl)bis (azanediyl))bis(carbonyl))bis(4-cyclopropylpyridine-6,3-diyl))bis(methyle ne))bis(azanediyl))bis(3-hydroxy-2-methylpropanoic acid)

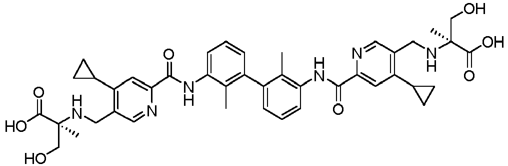

### Step 1: Synthesis of N,N'-(2,2'-dimethyl-[1,1'-biphenyl]-3,3'-diyl)bis(4-cyclopropyl-5-(dimethoxymethyl)picolin amide)

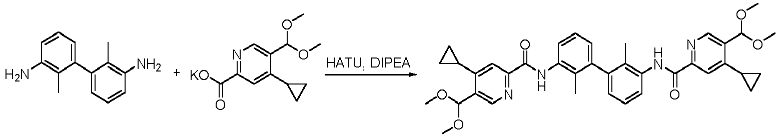

Potassium 4-cyclopropyl-5-(dimethoxymethyl)picolinate (2.80 g, 10.1 mmol), HATU (3.54 g, 9.24 mmol), 2,2'-dimethyl-[1,1'-diphenyl]-3,3'-diamine (700 mg, 3.3 mmol) and DIPEA (2.5 mL) were weighed into a 100 mL eggplant-shaped flask, DMF/THF (20 m L, v/v = 4/1) was added, and the mixture was stirred at room temperature for 6 hrs. T he reaction solution was filtered and washed. The filtrate was concentrated and separated by silica gel column chromatography to obtain *N*,*N*'-(2,2'-dimethyl-[1,1'-biphenyl]-3,3'-diy l)bis(4-cyclopropyl-5-(dimethoxymethyl)picolinamide) (1.30 g, yield: 61%). MS m/z (ESI): 651.6 [M+H]⁺.

### Step 2: Synthesis of N,N'-(2,2'-dimethyl-[1,1'-biphenyl]-3,3'-diyl)bis(4-cyclopropyl-5-formylpicolinamide)

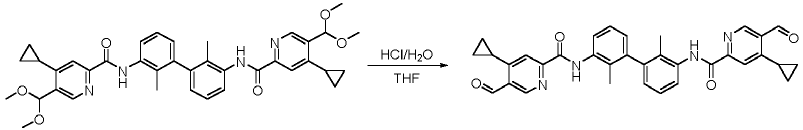

*N*,*N*'-(2,2'-dimethyl-[1,1'-biphenyl]-3,3'-diyl)bis(4-cyclopropyl-5-(dimethoxymethyl)p icolinamide) (1300 mg, 2.0 mmol) was added with THF (20 mL), and then the reaction solution was added with a hydrochloric acid solution (8 mL, 4 M) and was stirred at room temperature for 4 hrs. After the reaction was completed, the reaction solution was added with water (8 mL) and stirred overnight. The reaction solution was filtrated and dried in vacuum to obtain *N*,*N*'-(2,2'-dimethyl-[1,1'-biphenyl]-3,3'-diyl)bis(4-cyclopropyl-5-formylpicolinamide) (995 mg, yield: 90%). MS m/z (ESI): 559.2 [M+H]⁺.

### Step 3: Synthesis of (2S,2'S)-2,2'-((((((2,2'-dimethyl-[1,1'-biphenyl]-3,3'-diyl)bis(azanedi yl))bis(carbonyl))bis(4-cyclopropylpyridine-6,3-diyl))bis(methylene))bis(azanediyl))bis(3-hydroxy-2-methylpropanoic acid)

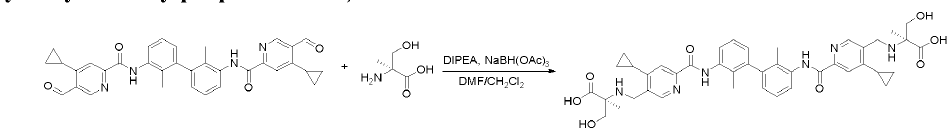

*N*,*N*'-(2,2'-dimethyl-[1,1'-biphenyl]-3,3'-diyl)bis(4-cyclopropyl-5-formylpicolinamide) (50 mg, 0.09 mmol) was added to DMF/CH₂Cl₂ (1.8 mL/0.9 mL), and then the reaction so lution was added with DIPEA (116 mg, 0.90 mmol) and (*S*)-2-amino-3-hydroxy-2-methyl propanoic acid (107 mg, 0.90 mmol), stirred at room temperature for 6 hrs, then added with NaBH (OAc)₃ (190 mg, 0.90 mmol) and stirred at room temperature overnight. Aft er the reaction was completed, the reaction solution was directly separated by reversed-p hase column chromatography (eluent : water (0.1% TFA) : acetonitrile), and lyophilized to obtain a TFA salt of (2*S*,2'*S*)-2,2'-((((((2,2'-dimethyl-[1,1'-biphenyl]-3,3'-diyl)bis(azanedi yl))bis(carbonyl))bis(4-cyclopropylpyridine-6,3-diyl))bis(methylene))bis(azanediyl))bis(3-hydr oxy-2-methylpropanoic acid) (17.2 mg, yield: 25%). MS m/z (ESI): 382.8 [1/2M+H]⁺; 76 5.7 [M+H]⁺.

¹H NMR (400 MHz, DMSO-*d*₆) δ 10.38 (s, 2H), 8.74 (s, 2H), 7.80 (d, *J* = 8.0 Hz, 2H), 7.67 (s, 2H), 7.34 (t, *J* = 8.0 Hz, 2H), 7.01 (d, *J* = 8.0 Hz, 2H), 4.54 - 4.46 (m, 4H), 3.97 (d, *J* = 12.0 Hz, 2H), 3.84 (d, *J* = 12.0 Hz, 2H), 2.43 - 2.36 (m, 4H), 2.00 (s, 6H), 1.56 (s, 6H), 1.24 - 1.16 (m, 4H), 0.96 - 0.91 (m, 4H).

Examples 68 to 70 were prepared by selecting corresponding raw materials according to all or part of the synthesis method in Example 67.

| Example No. | Structure | Name | MS: *m*/*z* [M+1]⁺ |
|---|---|---|---|
| 68 | 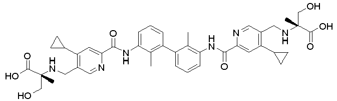 | (2*R*,2'*R*)-2,2'-((((((2,2'-dimethyl-[1,1' -biphenyl]-3,3'-diyl)bis(azanediyl))b is(carbonyl))bis(4-cyclopropylpyridi ne-6,3-diyl))bis(methylene))bis(azan ediyl))bis(3-hydroxy-2-methylpropa noic acid) | 765.7 |
| 69 | 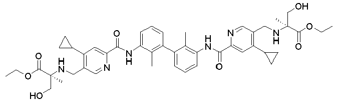 | diethyl 2,2'-((((((2,2'-dimethyl-[1,1'-biphenyl]-3,3'-diyl)bis(azanediyl))bi s(carbonyl))bis(4-cyclopropylpyridi ne-6,3-diyl))bis(methylene))bis(azan ediyl))(2*S*,2'*S*)-bis(3-hydroxy-2-met hylpropanoate) | 821.4 |
| 70 | 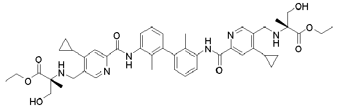 | diethyl 2,2'-((((((2,2'-dimethyl-[1,1'-biphenyl]-3,3'-diyl)bis(azanediyl))bi s(carbonyl))bis(4-cyclopropylpyridi ne-6,3-diyl))bis(methylene))bis(azan ediyl))(2*R*,2'*R*)-bis(3-hydroxy-2-met hylpropanoate) | 821.4 |

### ¹H NMR data of part of the compounds prepared in the above example are as follows:

| Example No. | ¹H-NMR |
|---|---|
| 2 | ¹H NMR (400 MHz, DMSO-*d*₆) δ 10.33 (s, 1H), 10.32 (s, 1H), 8.63 (s, 1H), 8.52 (s, 1H), 7.88 (d, *J* = 7.6 Hz, 2H), 7.62 (s, 1H), 7.59 (s, 1H), 7.32 (t, *J* = 7.9 Hz, 2H), 6.99 (dd, *J* = 7.7, 3.0 Hz, 2H), 4.22 - 3.99 (m, 3H), 3.71 (d, *J* = 13.6 Hz, 1H), 3.25 - 3.15 (m, 2H), 2.90 - 2.80 (m, 2H), 2.48 - 2.43 (m, 2H), 2.34 - 2.21 (m, 2H), 2.01 (s, 6H), 1.83 - 1.72 (m, 2H), 1.56 - 1.38 (m, 4H), 1.21 - 1.00 (m, 7H), 0.97 - 0.73 (m, 4H). |
| 3 | ¹H NMR (400 MHz, DMSO-*d*₆) δ 10.31 (s, 2H), 8.58 (s, 1H), 8.52 (s, 1H), 7.88 (dd, *J* = 8.1, 3.9 Hz, 2H), 7.59 (d, *J* = 2.9 Hz, 2H), 7.31 (t, *J* = 7.8 Hz, 2H), 6.98 (d, *J* = 7.5 Hz, 2H), 4.03 (d, *J* = 13.8 Hz, 2H), 3.95 (d, *J* = 13.8 Hz, 1H), 3.70 (d, *J* = 13.9 Hz, 1H), 3.18 (t, *J* = 5.7 Hz, 2H), 2.90 - 2.81 (m, 2H), 2.75 - 2.65 (m, 2H), 2.33 - 2.20 (m, 2H), 2.00 (s, 6H), 1.76 (s, 2H), 1.46 (brs, 4H), 1.12 (t, *J* = 8.5 Hz, 4H), 1.00 (d, *J* = 6.2 Hz, 3H), 0.94 - 0.74 (m, 4H). |
| 4 | ¹H NMR (400 MHz, DMSO-*d*₆) δ 10.31 (s, 2H), 8.60 (s, 1H), 8.52 (s, 1H), 7.88 (d, *J* = 8.0 Hz, 2H), 7.61 (s, 1H), 7.59 (s, 1H), 7.32 (t, *J* = 7.8 Hz, 2H), 6.98 (d, *J* = 7.5 Hz, 2H), 4.15 - 3.98 (m, 3H), 3.89 (s, 1H), 3.71 (d, *J* = 13.6 Hz, 1H), 3.19 (t, *J* = 5.6 Hz, 1H), 2.97 - 2.78 (m, 3H), 2.33 - 2.19 (m, 2H), 2.01 (s, 6H), 1.97 - 1.69 (m, 4H), 1.69 - 1.56 (m, 2H), 1.56 - 1.36 (m, 6H), 1.12 (t, *J* = 9.7 Hz, 4H), 0.95 - 0.72 (m, 4H). |
| 6 | ¹H NMR (400 MHz, DMSO-*d*₆) δ 10.31 (s, 2H), 8.58 (s, 1H), 8.51 (s, 1H), 7.88 (dd, *J* = 8.1, 5.2 Hz, 2H), 7.59 (s, 1H), 7.58 (s, 1H), 7.31 (t, *J* = 7.8 Hz, 2H), 6.97 (d, *J* = 7.5 Hz, 2H), 4.61 (s, 1H), 4.02 (d, *J* = 14.6 Hz, 3H), 3.84 (d, *J* = 3.6 Hz, 1H), 3.70 (d, *J* = 13.7 Hz, 2H), 3.19 (t, *J* = 5.7 Hz, 2H), 2.90 - 2.78 (m, 4H), 2.30 - 2.20 (m, 2H), 2.00 (s, 6H), 1.96 - 1.72 (m, 4H), 1.65 - 1.55 (m, 2H), 1.54 - 1.28 (m, 6H), 1.28 - 1.16 (m, 9H), 1.15 - 1.06 (m, 4H), 0.95 - 0.74 (m, 4H). |
| 7 | ¹H NMR (400 MHz, DMSO-*d*₆) δ 10.32 (s, 1H), 10.31 (s, 1H), 8.58 (s, 1H), 8.51 (s, 1H), 7.86 (t, *J* = 8.7 Hz, 2H), 7.60 (s, 1H), 7.58 (s, 1H), 7.31 (t, *J* = 7.8 Hz, 2H), 6.98 (dd, *J* = 7.6, 3.5 Hz, 2H), 4.07 (s, 2H), 4.03 (d, *J* = 13.7 Hz, 1H), 3.70 (d, *J* = 13.7 Hz, 1H), 3.22 - 3.15 (m, 1H), 2.89 - 2.80 (m, 2H), 2.45 (s, 3H), 2.32 - 2.22 (m, 2H), 2.00 (s, 6H), 1.81 - 1.72 (m, 2H), 1.52 - 1.38 (m, 2H), 1.22 - 1.05 (m, 6H), 0.94 - 0.72 (m, 4H). |
| 8 | ¹H NMR (400 MHz, DMSO-*d*₆) δ 10.32 (s, 1H), 10.31 (s, 1H), 8.60 (s, 1H), 8.51 (s, 1H), 7.86 (dd, *J* = 8.1, 4.6 Hz, 2H), 7.60 (s, 1H), 7.58 (s, 1H), 7.31 (t, *J* = 7.8 Hz, 2H), 6.98 (dd, *J* = 7.6, 3.4 Hz, 2H), 4.10 (s, 2H), 4.03 (d, *J* = 13.8 Hz, 1H), 3.70 (d, *J* = 13.7 Hz, 2H), 3.22 - 3.15 (m, 1H), 2.89 - 2.80 (m, 1H), 2.76 (q, *J* = 7.3 Hz, 2H), 2.47 - 2.42 (m, 1H), 2.34 - 2.20 (m, 2H), 2.00 (s, 6H), 1.81 - 1.73 (m, 2H), 1.52 - 1.43 (m, 2H), 1.22 - 1.09 (m, 7H), 0.88 (t, *J* = 5.3 Hz, 4H). |
| 9 | ¹H NMR (400 MHz, DMSO-*d*₆) δ 10.34 (s, 1H), 10.31 (s, 1H), 8.67 (s, 1H), 8.51 (s, 1H), 7.85 (t, *J* = 8.8 Hz, 2H), 7.64 (s, 1H), 7.58 (s, 1H), 7.32 (td, *J* = 7.8, 3.4 Hz, 2H), 6.98 (t, *J* = 7.6 Hz, 2H), 4.25 (s, 2H), 4.03 (d, *J* = 13.7 Hz, 1H), 3.71 (d, *J* = 13.8 Hz, 2H), 3.23 - 3.15 (m, 2H), 2.89 - 2.79 (m, 2H), 2.48 - 2.40 (m, 1H), 2.34 - 2.20 (m, 2H), 2.00 (s, 3H), 1.99 (s, 3H), 1.81 - 1.70 (m, 2H), 1.54 - 1.37 (m, 4H), 1.22 (d, *J* = 6.2 Hz, 6H), 1.22 - 1.07 (m, 4H), 0.96 - 0.71 (m, 4H). |
| 10 | ¹H NMR (400 MHz, DMSO-*d*₆) δ 10.35 (s, 1H), 10.31 (s, 1H), 8.71 (s, 1H), 8.52 (s, 1H), 7.87 (d, *J* = 8.0 Hz, 1H), 7.82 (d, *J* = 8.0 Hz, 1H), 7.64 (s, 1H), 7.59 (s, 1H), 7.32 (td, *J* = 7.8, 3.2 Hz, 2H), 6.99 (t, *J* = 8.4 Hz, 2H), 4.45 (s, 2H), 4.03 (d, *J* = 13.6 Hz, 1H), 3.72 (d, *J* = 13.8 Hz, 1H), 3.24 - 3.17 (m, 2H), 2.91 - 2.80 (m, 1H), 2.48 - 2.42 (m, 1H), 2.35 - 2.20 (m, 2H), 2.00 (d, *J* = 3.8 Hz, 6H), 1.80 - 1.72 (m, 2H), 1.53 - 1.43 (m, 4H), 1.25 - 1.15 (m, 2H), 1.15 - 1.08 (m, 2H), 0.98 - 0.85 (m, 4H), 0.84 - 0.65 (m, 4H). |
| 11 | ¹H NMR (400 MHz, DMSO-*d*₆) δ 10.33 (s, 1H), 10.31 (s, 1H), 8.65 (s, 1H), 8.52 (s, 1H), 7.86 (t, *J* = 7.8 Hz, 2H), 7.63 (s, 1H), 7.59 (s, 1H), 7.32 (td, *J* = 7.8, 3.4 Hz, 2H), 6.98 (t, *J* = 7.4 Hz, 2H), 4.20 (brs, 2H), 4.03 (d, *J* = 13.8 Hz, 1H), 3.71 (d, *J* = 13.7 Hz, 1H), 3.20 (t, *J* = 5.5 Hz, 1H), 2.90 - 2.80 (m, 1H), 2.35 - 2.20 (m, 2H), 2.01 (s, 3H), 2.00 (s, 3H), 1.95 - 1.85 (m, 2H), 1.80 - 1.65 (m, 4H), 1.60 - 1.40 (m, 8H), 1.20 - 1.05 (m, 4H), 0.95 - 0.75 (m, 4H). |
| 12 | ¹H NMR (400 MHz, DMSO-*d*₆) δ 10.30 (s, 2H), 8.53 (s, 1H), 8.52 (s, 1H), 7.88 (d, *J* = 8.0 Hz, 2H), 7.59 (s, 2H), 7.32 (t, *J* = 7.8 Hz, 2H), 6.98 (d, *J* = 7.5 Hz, 2H), 4.03 (d, *J* = 13.7 Hz, 1H), 3.89 (brs, 2H), 3.71 (d, *J* = 13.8 Hz, 2H), 3.20 (t, *J* = 5.6 Hz, 1H), 2.90 - 2.81 (m, 1H), 2.56 (brs, 2H), 2.49 - 2.40 (m, 1H), 2.41 - 2.30 (m, 2H), 2.31 - 2.21 (m, 1H), 2.01 (s, 6H), 1.82 - 1.70 (m, 6H), 1.56 - 1.40 (m, 4H), 1.20 - 1.07 (m, 4H), 0.91 - 0.74 (m, 4H). |
| 13 | ¹H NMR (400 MHz, DMSO-*d*₆) δ 10.31 (s, 2H), 8.52 (s, 2H), 7.88 (d, *J* = 8.4 Hz, 2H), 7.59 (s, 2H), 7.32 (t, *J* = 7.8 Hz, 2H), 6.98 (d, *J* = 7.5 Hz, 2H), 4.71 (brs, 1H), 4.21 (brs, 1H), 4.03 (d, *J* = 13.8 Hz, 1H), 3.86 (d, *J* = 13.4 Hz, 1H), 3.80 (d, *J* = 13.6 Hz, 1H), 3.70 (d, *J* = 13.7 Hz, 1H), 3.21 - 3.17 (m, 1H), 2.98 - 2.81 (m, 4H), 2.75 (dd, *J* = 9.5, 6.2 Hz, 1H), 2.42 - 2.30 (m, 2H), 2.31 - 2.20 (m, 2H), 2.01 (s, 6H), 1.82 - 1.71 (m, 2H), 1.52 - 1.37 (m, 4H), 1.18 - 1.05 (m, 6H), 0.95 - 0.73 (m, 4H). |
| 14 | ¹H NMR (400 MHz, DMSO-*d*₆) δ 10.30 (s, 2H), 8.52 (s, 2H), 7.89 (d, *J* = 8.1 Hz, 2H), 7.59 (s, 1H), 7.58 (s, 1H), 7.31 (t, *J* = 7.8 Hz, 2H), 6.98 (d, *J* = 7.4 Hz, 2H), 4.21 (tt, *J* = 7.0, 3.5 Hz, 1H), 4.05 (d, *J* = 13.5 Hz, 1H), 3.86 (d, *J* = 13.5 Hz, 1H), 3.80 (d, *J* = 13.6 Hz, 1H), 3.67 (d, *J* = 14.0 Hz, 1H), 3.13 (brs, 2H), 3.00 - 2.80 (m, 2H), 2.75 (dd, *J* = 9.6, 6.2 Hz, 1H), 2.70 - 2.62 (m, 1H), 2.43 - 2.30 (m, 3H), 2.22 (brs, 1H), 2.05 - 1.95 (m, 1H), 2.01 (s, 6H), 1.80 - 1.70 (m, 2H), 1.63 - 1.32 (m, 6H), 1.20 - 1.07 (m, 4H), 0.90 - 0.70 (m, 4H). |
| 15 | ¹H NMR (400 MHz, DMSO-*d*₆) δ 10.31 (s, 2H), 8.52 (s, 2H), 7.86 (t, *J* = 8.3 Hz, 2H), 7.60 (s, 1H), 7.59 (s, 1H), 7.32 (t, *J* = 7.8 Hz, 2H), 6.98 (dd, *J* = 7.7, 3.4 Hz, 2H), 4.03 (d, *J* = 13.8 Hz, 2H), 3.71 (d, *J* = 13.7 Hz, 2H), 3.20 (t, *J* = 5.6 Hz, 4H), 2.90 - 2.81 (m, 2H), 2.45 (d, *J* = 7.6 Hz, 1H), 2.32 - 2.20 (m, 2H), 2.00 (s, 6H), 1.82 - 1.72 (m, 2H), 1.53 - 1.43 (m, 4H), 1.40 (s, 3H), 1.20 - 1.07 (m, 4H), 0.95 - 0.73 (m, 4H). |
| 23 | ¹H NMR (400 MHz, DMSO-*d*₆) δ 10.34 (s, 1H), 10.31 (s, 1H), 8.65 (s, 1H), 8.52 (s, 1H), 7.85 (dd, *J* = 12.7, 8.1 Hz, 2H), 7.61 (s, 1H), 7.58 (s, 1H), 7.32 (td, *J* = 7.8, 2.8 Hz, 2H), 6.98 (t, *J* = 7.4 Hz, 2H), 4.70 (t, *J* = 7.1 Hz, 2H), 4.54 (brs, 2H), 4.23 (brs, 2H), 4.05 (d, *J* = 13.6 Hz, 1H), 3.74 (d, *J* = 12.9 Hz, 1H), 2.90 - 2.80 (m, 2H), 2.48 - 2.38 (m, 1H), 2.38 - 2.21 (m, 2H), 2.00 (d, *J* = 2.7 Hz, 6H), 1.83 - 1.71 (m, 2H), 1.56 - 1.38 (m, 4H), 1.23 - 1.08 (m, 4H), 0.98 - 0.73 (m, 4H). |
| 24 | ¹H NMR (400 MHz, DMSO-*d*₆) δ 10.33 (s, 1H), 10.31 (s, 1H), 8.66 (s, 1H), 8.51 (s, 1H), 7.86 (t, *J* = 6.9 Hz, 2H), 7.63 (s, 1H), 7.58 (s, 1H), 7.32 (td, *J* = 7.9, 2.8 Hz, 2H), 6.98 (t, *J* = 6.8 Hz, 2H), 4.22 (brs, 2H), 4.03 (d, *J* = 13.7 Hz, 1H), 3.90 (d, *J* = 11.7 Hz, 2H), 3.71 (d, *J* = 13.7 Hz, 1H), 3.19 (t, *J* = 5.7 Hz, 2H), 2.89 - 2.80 (m, 2H), 2.48 - 2.40 (m, 2H), 2.35 - 2.20 (m, 2H), 2.00 (d, *J* = 2.4 Hz, 6H), 1.98 - 1.90 (m, 2H), 1.80 - 1.70 (m, 2H), 1.55 - 1.34 (m, 4H), 1.20 - 1.05 (m, 4H), 0.96 - 0.70 (m, 4H). |
| 25 | ¹H NMR (400 MHz, DMSO-*d*₆) δ 10.31 (s, 2H), 8.57 (s, 1H), 8.52 (s, 1H), 7.89 (dd, *J* = 8.1, 4.7 Hz, 2H), 7.59 (s, 1H), 7.58 (s, 1H), 7.31 (t, *J* = 7.8 Hz, 2H), 6.98 (d, *J* = 7.5 Hz, 2H), 4.04 (d, *J* = 13.7 Hz, 1H), 3.96 (d, *J* = 13.8 Hz, 1H), 3.91 (d, *J* = 13.8 Hz, 1H), 3.82 - 3.63 (m, 5H), 3.52 - 3.44 (m, 2H), 3.16 (t, *J* = 5.7 Hz, 1H), 2.89 - 2.80 (m, 1H), 2.34 - 2.19 (m, 2H), 2.00 (s, 6H), 2.00 - 1.92 (m, 1H), 1.81 - 1.70 (m, 3H), 1.54 - 1.36 (m, 4H), 1.18 - 1.07 (m, 4H), 0.94 - 0.74 (m, 4H). |
| 27 | ¹H NMR (400 MHz, DMSO-*d*₆) δ 10.30 (s, 2H), 8.54 (s, 1H), 8.52 (s, 1H), 7.89 (t, *J* = 8.7 Hz, 2H), 7.59 (s, 1H), 7.58 (s, 1H), 7.32 (t, *J =* 7.8 Hz, 2H), 6.98 (d, *J* = 7.5 Hz, 2H), 4.34 (d, *J* = 13.4 Hz, 1H), 4.03 (d, *J* = 13.7 Hz, 1H), 3.70 (d, *J* = 13.5 Hz, 1H), 3.61 (d, *J* = 13.4 Hz, 1H), 3.50 - 3.40 (m, 2H), 3.20 - 3.15 (m, 1H), 2.91 - 2.81 (m, 1H), 2.81 - 2.62 (m, 4H), 2.30 - 2.20 (m, 2H), 2.01 (s, 6H), 1.93 - 1.82 (m, 1H), 1.80 - 1.75 (m, 1H), 1.70 - 1.55 (m, 4H), 1.50 - 1.35 (m, 4H), 1.15 - 1.00 (m, 4H), 0.99 0.84 (m, 2H), 0.84 - 0.70 (m, 2H). |
| 28 | ¹H NMR (400 MHz, DMSO-*d*₆) δ 10.30 (s, 2H), 8.54 (s, 1H), 8.52 (s, 1H), 7.89 (t, *J* = 8.3 Hz, 2H), 7.59 (s, 1H), 7.58 (s, 1H), 7.31 (t, *J* = 7.8 Hz, 2H), 6.98 (d, *J* = 7.5 Hz, 2H), 4.35 (d, *J* = 13.4 Hz, 1H), 4.03 (d, *J* = 13.7 Hz, 1H), 3.71 (d, *J* = 13.6 Hz, 1H), 3.67 - 3.55 (m, 1H), 3.50 - 3.40 (m, 1H), 3.23 - 3.06 (m, 3H), 2.91 - 2.75 (m, 2H), 2.47 - 2.37 (m, 2H), 2.35 - 2.20 (m, 2H), 2.00 (s, 6H), 1.94 - 1.81 (m, 1H), 1.80 - 1.74 (m, 1H), 1.71 - 1.54 (m, 2H), 1.50 - 1.35 (m, 4H), 1.17 - 1.05 (m, 4H), 0.99 - 0.84 (m, 2H), 0.82 - 0.72 (m, 2H). |
| 31 | ¹H NMR (400 MHz, DMSO-*d*₆) δ 10.31 (s, 1H), 10.30 (s, 1H), 8.55 (s, 1H), 8.52 (s, 1H), 7.93 - 7.84 (m, 2H), 7.59 (s, 2H), 7.32 (t, *J* = 7.8 Hz, 2H), 6.98 (d, *J* = 7.7 Hz, 2H), 4.11 (q, *J* = 7.6 Hz, 2H), 4.03 (d, *J* = 13.6 Hz, 2H), 3.72 (d, *J* = 13.7 Hz, 1H), 3.62 (brs, 2H), 2.90 - 2.82 (m, 2H), 2.68 - 2.65 (m, 1H), 2.35 - 2.23 (m, 3H), 2.01 (s, 3H), 2.00 (s, 3H), 1.82 - 1.72 (m, 2H), 1.55 - 1.40 (m, 4H), 1.20 (t, *J* = 7.1 Hz, 3H), 1.15 - 1.07 (m, 4H), 0.95 - 0.75 (m, 4H). |
| 32 | ¹H NMR (400 MHz, DMSO-*d*₆) δ 10.35 (s, 1H), 10.32 (s, 1H), 8.74 (s, 1H), 8.54 (s, 1H), 7.87 (d, *J* = 8.0 Hz, 1H), 7.83 (d, *J* = 8.0 Hz, 1H), 7.64 (s, 1H), 7.60 (s, 1H), 7.33 (t, *J* = 7.7 Hz, 2H), 6.99 (t, *J* = 7.1 Hz, 2H), 4.38 (brs, 2H), 4.07 (d, *J* = 13.7 Hz, 1H), 4.00 - 3.82 (m, 2H), 3.76 (d, *J* = 13.8 Hz, 1H), 2.90 - 2.80 (m, 1H), 2.41 - 2.26 (m, 2H), 2.01 (s, 3H), 2.00 (s, 3H), 1.86 - 1.74 (m, 2H), 1.57 - 1.37 (m, 4H), 1.21 - 1.08 (m, 4H), 0.98 - 0.73 (m, 4H). |
| 37-1 | ¹H NMR (400 MHz, DMSO-*d*₆) δ 10.31 (s, 2H), 8.58 (s, 1H), 8.54 (s, 1H), 7.88 (d, *J* = 8.1 Hz, 2H), 7.60 (s, 1H), 7.59 (s, 1H), 7.32 (t, *J* = 7.8 Hz, 2H), 6.98 (d, *J* = 7.5 Hz, 3H), 4.85 (t, *J* = 5.9 Hz, 1H), 4.58 (brs, 1H), 4.10 (q, *J* = 7.2 Hz, 2H), 4.06 (d, *J* = 13.6 Hz, 1H), 4.03 (s, 2H), 3.90 (d, *J* = 13.7 Hz, 1H), 3.61 (t, *J* = 5.3 Hz, 2H), 3.52 (t, *J* = 5.3 Hz, 2H), 3.37 - 3.33 (m, 1H), 2.70 (t, *J* = 5.7 Hz, 2H), 2.37 - 2.23 (m, 2H), 2.00 (s, 6H), 1.20 (t, *J* = 7.1 Hz, 3H), 1.18 - 1.06 (m, 4H), 0.90 - 0.78 (m, 4H). |
| 37 | ¹H NMR (400 MHz, DMSO-*d*₆) δ 10.32 (s, 1H), 10.31 (s, 1H), 8.582 (s, 1H), 8.577 (s, 1H), 7.88 (dd, *J* = 8.1, 4.4 Hz, 2H), 7.60 (s, 2H), 7.31 (t, *J* = 7.8 Hz, 2H), 6.98 (d, *J* = 7.5 Hz, 2H), 4.14 (d, *J* = 13.7 Hz, 1H), 4.02 (s, 2H), 3.98 (d, *J* = 13.8 Hz, 1H), 3.64 (t, *J* = 5.0 Hz, 2H), 3.52 (t, *J* = 5.7 Hz, 2H), 3.28 - 3.21 (m, 1H), 2.74 - 2.65 (m, 2H), 2.35 - 2.24 (m, 2H), 2.00 (s, 6H), 1.21 - 1.08 (m, 4H), 0.94 - 0.81 (m, 4H). |
| 38-1 | ¹H NMR (400 MHz, DMSO-*d*₆) δ 10.32 (s, 1H), 10.31 (s, 1H), 8.62 (s, 1H), 8.54 (s, 1H), 7.86 (dd, *J* = 8.1, 3.4 Hz, 2H), 7.61 (s, 1H), 7.58 (s, 1H), 7.32 (t, *J* = 7.8 Hz, 2H), 6.98 (dd, *J* = 7.5, 3.1 Hz, 2H), 4.20 - 4.01 (m, 5H), 3.90 (d, *J* = 13.7 Hz, 1H), 3.61 (t, *J* = 4.7 Hz, 2H), 2.95 - 2.85 (s, 1H), 2.34 - 2.23 (m, 2H), 2.00 (s, 6H), 1.19 (t, *J* = 7.1 Hz, 3H), 1.18 - 1.08 (m, 4H), 1.09 (d, *J* = 6.5 Hz, 3H), 0.92 - 0.81 (m, 4H). |
| 38 | ¹H NMR (400 MHz, DMSO-*d*₆) δ 10.32 (s, 2H), 8.60 (s, 1H), 8.59 (s, 1H), 7.88 (t, *J* = 6.6 Hz, 2H), 7.60 (s, 2H), 7.32 (t, *J* = 7.8 Hz, 2H), 6.98 (d, *J* = 7.5 Hz, 2H), 4.15 (d, *J* = 13.7 Hz, 1H), 4.08 (d, *J* = 13.7 Hz, 1H), 4.00 (dd, *J* = 13.8, 4.3 Hz, 2H), 3.65 (t, *J* = 5.6 Hz, 2H), 3.40 - 3.20 (m, 3H), 2.77 (q, *J* = 6.2 Hz, 1H), 2.38 - 2.23 (m, 2H), 2.00 (s, 6H), 1.13 (d, *J* = 8.4 Hz, 4H), 1.03 (d, *J* = 6.3 Hz, 3H), 0.90 0.80 (m, 4H). |
| 39-1 | ¹H NMR (400 MHz, DMSO-*d*₆) δ 10.31 (s, 2H), 8.59 (s, 1H), 8.54 (s, 1H), 7.89 (dd, *J* = 8.3, 3.6 Hz, 2H), 7.60 (s, 1H), 7.59 (s, 1H), 7.32 (t, *J* = 7.8 Hz, 2H), 6.98 (d, *J* = 7.5 Hz, 2H), 4.14 - 4.02 (m, 4H), 3.97 (d, *J* = 13.8 Hz, 1H), 3.90 (d, *J* = 13.7 Hz, 1H), 3.62 (d, *J* = 4.9 Hz, 2H), 3.39 - 3.33 (m, 3H), 2.77 - 2.67 (m, 1H), 2.34 - 2.23 (m, 2H), 2.01 (s, 6H), 1.20 (t, *J* = 7.1 Hz, 3H), 1.17 - 1.08 (m, 4H), 1.01 (d, *J* = 6.4 Hz, 3H), 0.91 - 0.78 (m, 4H). |
| 39 | ¹H NMR (400 MHz, DMSO-*d*₆) δ 10.32 (s, 1H), 10.31 (s, 1H), 8.59 (s, 2H), 7.88 (t, *J* = 7.8 Hz, 2H), 7.60 (s, 2H), 7.32 (t, *J* = 7.8 Hz, 2H), 6.98 (d, *J* = 7.5 Hz, 2H), 4.14 (d, *J=* 13.8 Hz, 1H), 4.05 (d, *J=* 13.8 Hz, 1H), 3.98 (dd, *J=* 13.8, 4.2 Hz, 2H), 3.68 - 3.59 (m, 2H), 3.39 - 3.28 (m, 2H), 3.25 (t, *J* = 5.1 Hz, 1H), 2.80 - 2.65 (m, 1H), 2.39 - 2.24 (m, 2H), 2.00 (s, 6H), 1.13 (d, *J* = 8.5 Hz, 4H), 1.01 (d, *J* = 6.3 Hz, 3H), 0.95 - 0.80 (m, 4H). |
| 40 | ¹H NMR (400 MHz, DMSO-*d*₆) δ 10.31 (s, 1H), 10.30 (s, 1H), 8.58 (s, 1H), 8.57 (s, 1H), 7.87 (t, *J* = 8.8 Hz, 2H), 7.59 (s, 2H), 7.31 (t, *J* = 7.8 Hz, 2H), 6.97 (d, *J* = 7.6 Hz, 2H), 4.11 (d, *J* = 13.8 Hz, 1H), 3.97 (brs, 3H), 3.82 (q, *J* = 5.1 Hz, 1H), 3.70 - 3.55 (m, 2H), 3.12 - 3.04 (m, 1H), 2.82 (q, *J* = 5.7 Hz, 1H), 2.38 - 2.22 (m, 2H), 2.00 (s, 6H), 1.90 - 1.75 (m, 2H), 1.65 - 1.50 (m, 2H), 1.46 - 1.25 (m, 2H), 1.18 - 1.05 (m, 4H), 0.90 - 0.80 (m, 4H). |
| 51 | ¹H NMR (400 MHz, DMSO-*d*₆) δ 10.32 (s, 1H), 10.30 (s, 1H), 8.58 (s, 2H), 7.88 (t, *J* = 8.5 Hz, 2H), 7.59 (s, 2H), 7.31 (t, *J* = 7.8 Hz, 2H), 6.98 (d, *J* = 7.5 Hz, 2H), 4.14 (d, *J* = 13.8 Hz, 1H), 4.00 - 3.94 (m, 3H), 3.84 (dt, *J* = 11.6, 3.6 Hz, 2H), 3.68 - 3.58 (m, 2H), 3.30 - 3.20 (m, 3H), 2.73 - 2.64 (m, 1H), 2.36 - 2.25 (m, 2H), 2.00 (s, 6H), 1.84 (d, *J=* 11.9 Hz, 2H), 1.40 - 1.26 (m, 2H), 1.19 - 1.08 (m, 4H), 0.90 - 0.81 (m, 4H). |
| 53-1 | ¹H NMR (400 MHz, DMSO-*d*₆) δ 10.31 (s, 2H), 8.54 (s, 2H), 7.88 (d, *J* = 8.0 Hz, 2H), 7.59 (s, 2H), 7.32 (t, *J* = 7.8 Hz, 2H), 6.98 (d, *J* = 7.5 Hz, 2H), 4.85 (t, *J* = 5.8 Hz, 1H), 4.71 (brs, 1H), 4.23 (brs, 1H), 4.10 (q, *J* = 7.0 Hz, 2H), 4.06 (d, *J* = 13.6 Hz, 1H), 3.90 (d, *J* = 13.7 Hz, 1H), 3.84 (brs, 1H), 3.61 (t, *J* = 5.4 Hz, 2H), 3.39 - 3.32 (m, 1H), 2.85 - 2.65 (m, 2H), 2.39 - 2.24 (m, 2H), 2.10 - 1.95 (m, 1H), 2.01 (s, 6H), 1.65 - 1.52 (m, 1H), 1.20 (t, *J* = 7.1 Hz, 3H), 1.19 - 1.07 (m, 4H), 0.90 - 0.78 (m, 4H). |
| 53 | ¹H NMR (400 MHz, DMSO-*d*₆) δ 10.31 (s, 1H), 10.30 (s, 1H), 8.58 (s, 1H), 8.52 (s, 1H), 7.88 (t, *J=* 7.0 Hz, 2H), 7.60 (s, 1H), 7.59 (s, 1H), 7.31 (t, *J=* 7.8 Hz, 2H), 6.98 (d, *J* = 7.5 Hz, 2H), 4.26 - 4.16 (m, 1H), 4.14 (d, *J* = 13.8 Hz, 1H), 3.98 (d, *J* = 13.7 Hz, 1H), 3.86 (d, *J* = 13.6 Hz, 1H), 3.80 (d, *J* = 13.5 Hz, 1H), 3.68 - 3.58 (m, 2H), 2.75 (dd, *J* = 9.6, 6.1 Hz, 1H), 2.66 (q, *J* = 7.8 Hz, 1H), 2.39 (dd, *J* = 9.6, 3.5 Hz, 1H), 2.39 - 2.26 (m, 2H), 2.00 (s, 6H), 2.06 - 1.94 (m, 1H), 1.63 - 1.50 (m, 1H), 1.18 - 1.04 (m, 4H), 0.90 - 0.80 (m, 4H). |
| 54 | ¹H NMR (400 MHz, DMSO-*d*₆) δ 10.32 (s, 1H), 10.30 (s, 1H), 8.58 (s, 1H), 8.54 (s, 1H), 7.89 (dd, *J* = 11.8, 8.1 Hz, 2H), 7.59 (s, 1H), 7.58 (s, 1H), 7.32 (t, *J* = 7.8 Hz, 2H), 6.98 (d, *J* = 7.5 Hz, 2H), 4.34 (d, *J* = 13.3 Hz, 1H), 4.11 (d, *J* = 13.8 Hz, 1H), 3.96 (d, *J* = 13.8 Hz, 1H), 3.70 - 3.55 (m), 3.34 - 3.27 (m), 3.05 (t, *J* = 6.0 Hz, 1H), 2.77 (t, *J* = 7.8 Hz, 1H), 2.70 - 2.62 (m, 1H), 2.49 - 2.40 (m, 1H), 2.36 - 2.23 (m, 2H), 2.01 (s, 6H), 1.93 - 1.82 (m, 1H), 1.70 - 1.52 (m, 3H), 1.20 - 1.06 (m, 4H), 0.99 - 0.71 (m, 4H). |

### Biological Test Evaluation

### 1. PD1-PDL1 HTRF Binding Activity Test

The effect of compounds of the examples of the present invention on the interaction between PD-1 and PD-L1 was determined by the PD-1/PD-L1 binding assay kit from Cisbio (#64ICP01PEG or 64ICP01PEH). The detailed experimental process was as follows:
1) Pre-diluted compound solution, 4 µL of Tag1-PD-L1 and 4 µL of Tag2-PD1 were added to each well of a 384-well plate;
2) After the mixture was incubated at room temperature for 15 min, 5 µL of anti-Tag1-Eu3+ antibody and 5 µL of anti-Tag2-XL665 antibody were then added;
3) After incubated for 2 hrs at room temperature or overnight at 4 °C, plates were read on Envision of Perkin Elmer; and readings at 665 nm and 620 nm were recorded, and the ratio of the two readings was taken as a reading for each well;
4) The reading of each well after compound treatment was compared with the reading of DMSO treated wells to obtain the percent inhibition of the compound; and
5) IC₅₀ values of compounds of the examples of the present invention were determined by non-linear regression analysis of percent inhibition at different compound concentrations. The specific experimental results were shown in Table 1.

### 2. Jurkat Reporter Gene Cellular Assay

The effect of compounds of the examples of the present invention on the interaction between PD-1 and PD-L1 expressed on cell surfaces, and the related influence on T cell functions, were determined by a Jurkat reporter gene cellular assay.

Briefly, the reporter gene plasmid of NF-κB-luc and the plasmid of human PD-1 were transfected into Jurkat cells to establish a stably transfected cell line capable of stably expressing both PD-1 and NF-κB-Luc reporter genes; the expression level of PD-1 on the cell surface was confirmed by flow cytometry; and the expression of the reporter gene was confirmed via the response of the reporter gene stimulated by OKT-3 and Raji cells.

In addition, the plasmid of human PD-L1 was transfected into Raji cells to obtain a cell line capable of stably expressing PD-L1. Jurkat/NF-κB-luc/PD1 cells and Raji-PD-L1 cells were then cocultured and stimulated with OKT-3. On this basis, the compounds were added, and the enhancement of the signal pathway of T cell activation by the inhibitory effect of the compounds on the interaction between PD-1 and PD-L1 was evaluated by readings of reporter gene responses. The specific experimental process was as follows:
1) 30 µL of compound or antibody solution was added to each well of a white 96-well plate (Corning, 3610) at different diluted concentrations, and 10 µL of OKT3 (Biolegend, 317326) was then added (the final concentration of OKT3: 1 µg/mL);
2) 20 µL of Raji-PD-L1 cell suspension was added to each well with 5 ^{∗} 10⁴ cells for each well, and was incubated in an incubator for 20 min;
3) 20 µL of Jurkat/NF-κb-luc/PD-1 cell suspension was added to each well with 5 ^{∗} 10⁴ cells for each well, and was well mixed, and 6 hrs later, Bright-glo (Promega, E2620) was applied and plates were read on Envision;
4) The reading of each well treated with the compound was compared with the reading of each well treated with DMSO to obtain the activation fold of the compound; and
5) EC₅₀ values of compounds of the examples of the present invention were determined by non-linear regression analysis of activation folds at different compound concentrations. The specific experimental results were shown in Table 1:

**Table 1: Biological Test Results**

| Example No. | PD1-PDL1 HTRF Binding Activity IC₅₀/nM | Cell Activity EC₅₀/nM | Example No. | PD1-PDL1 HTRF Binding Activity IC₅₀/nM | Cell Activity EC₅₀/nM |
|---|---|---|---|---|---|
| 1 | 1.07 | 6.8 | 37-1 | 5.58 | 265.2 |
| 2 | 1.21 | 9.2 | 37 | 0.95 | 25.2 |
| 3 | 1.07 | 14.9 | 38-1 | 4.77 | 219.4 |
| 4 | 1.02 | 3.2 | 38 | 0.67 | 37.3 |
| 5 | NT | NT | 39-1 | 3.22 | 264.2 |
| 6 | 1.69 | 49.3 | 39 | 1.89 | 36.0 |
| 7 | 0.49 | 24.1 | 40 | 1.68 | 39.2 |
| 8 | 1.27 | 23.8 | 41 | NT | NT |
| 9 | 5.10 | 33.9 | 42 | NT | NT |
| 10 | 3.38 | 119.5 | 43 | NT | NT |
| 11 | 3.19 | 14.5 | 44 | NT | NT |
| 12 | 1.02 | 10.6 | 45 | NT | NT |
| 13 | 1.48 | 11.8 | 46 | NT | NT |
| 14 | 1.76 | 8.2 | 47 | NT | NT |
| 15 | 0.68 | 15.1 | 48 | NT | NT |
| 16 | NT | NT | 49 | NT | NT |
| 17 | NT | NT | 50 | NT | NT |
| 18 | NT | NT | 51 | 2.05 | 87.1 |
| 19 | NT | NT | 52 | NT | NT |
| 20 | NT | NT | 53-1 | 5.77 | 258.5 |
| 21 | NT | NT | 53 | 3.61 | 86.8 |
| 22 | NT | NT | 54 | 0.91 | 62.3 |
| 23 | 2.57 | 77.5 | 55 | 3.18 | 99.5 |
| 24 | 0.91 | 52.0 | 56 | NT | NT |
| 25 | 0.91 | 8.7 | 57 | NT | NT |
| 26 | NT | NT | 58 | NT | NT |
| 27 | 2.62 | 27.3 | 59 | NT | NT |
| 28 | 4.17 | 35.3 | 60 | NT | NT |
| 29 | 1.61 | 115.2 | 61 | NT | NT |
| 30 | 0.97 | 18.1 | 62 | NT | NT |
| 31 | 2.57 | 42.6 | 63 | NT | NT |
| 32 | 1.85 | 44.8 | 64 | NT | NT |
| 33 | NT | NT | 65 | NT | NT |
| 34 | NT | NT | 66 | NT | NT |
| 35-1 | 3.03 | 231.4 | 67 | 0.61 | 19.7 |
| 35 | 1.95 | 28.6 | 68 | NT | NT |
| 36-1 | 64.3 | NT | 69 | NT | NT |
| 36 | 0.72 | 52 | 70 | NT | NT |
| Notes | "NT" is an abbreviation of "Not Tested", and means that an object has not been detected yet. | | | | |

The bioactivity data of the compounds of the specific examples indicates that the series of compounds of the present invention have a strong inhibitory effect on the interaction between PD-1 and PD-L1, and moreover, such an inhibitory effect can enhance or recover the activation of T cells at the cellular level.

All documents mentioned in the present invention are incorporated as references, just as each document is individually cited as a reference. In addition, it should be understood that various modifications or changes may be made by those skilled in the art after reading the above teachings of the present invention, and these equivalent forms also fall within the scope defined by the claims appended hereto.

## Claims

1. A compound of formula (I), a stereoisomer, prodrug or pharmaceutically acceptable salt thereof: 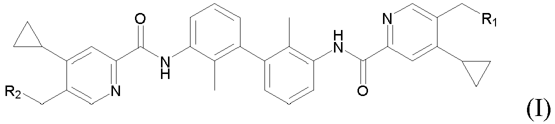 wherein,
R₁ is selected from the group consisting of 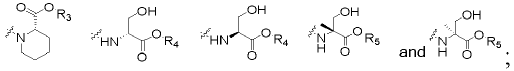
R₂ is -NHR₆ or 4-6 membered nitrogen-containing heterocyclyl, wherein a nitrogen atom is linked to methylene, and the 4-6 membered nitrogen-containing heterocyclyl is optionally further substituted with one or more substituents selected from the group consisting of deuterium, halogen, cyano, C₁₋₄ alkyl, C₁₋₄ haloalkyl, hydroxy-substituted C₁₋₄ alkyl, C₁₋₄ deuterioalkyl, C₃₋₆ cycloalkyl, 4-6 membered heterocyclyl, hydroxy-substituted C₃₋₆ cycloalkyl, carboxy-substituted C₃₋₆ cycloalkyl, hydroxy-substituted 4-6 membered heterocyclyl, carboxy-substituted 4-6 membered heterocyclyl, hydroxy, C₁₋₄ alkoxy, carboxy and C₁₋₄ alkoxyacyl;
R₃, R₄ and R₅ are each independently selected from the group consisting of hydrogen, deuterium and C₁₋₄ alkyl, the C₁₋₄ alkyl is optionally further substituted with one or more substituents selected from the group consisting of deuterium, halogen, cyano, cyclopropyl, hydroxy and C₁₋₄ alkoxy;
R₆ is selected from the group consisting of C₁₋₄ alkyl, C₃₋₆ cycloalkyl and C₄₋₆ heterocyclyl, the heterocyclyl optionally contains 1-2 heteroatoms selected from N and O, above groups are optionally further substituted with one or more substituents selected from the group consisting of deuterium, halogen, cyano, C₁₋₄ alkyl, C₁₋₄ haloalkyl, hydroxy-substituted C₁₋₄ alkyl, C₁₋₄ deuterioalkyl, C₃₋₆ cycloalkyl, 4-6 membered heterocyclyl, hydroxy-substituted C₃₋₆ cycloalkyl, carboxy-substituted C₃₋₆ cycloalkyl, hydroxy-substituted 4-6 membered heterocyclyl, carboxy-substituted 4-6 membered heterocyclyl, hydroxy, C₁₋₄ alkoxy, carboxy and C₁₋₄ alkoxyacyl;
provided that when R₁ is 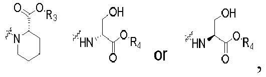 R₂ is different from R₁.

2. The compound of formula (I), the stereoisomer, prodrug or pharmaceutically acceptable salt thereof according to claim 1, wherein,
R₃, R₄ and R₅ are each independently selected from the group consisting of hydrogen, deuterium, methyl, ethyl and propyl, above groups are optionally further substituted with one or more substituents selected from the group consisting of deuterium, fluoro, chloro, bromo, cyano, cyclopropyl, hydroxy, methoxy and ethoxy;
R₆ is selected from the group consisting of methyl, ethyl, isopropyl, cyclopropyl, cyclobutyl, cyclopentyl, cyclohexyl, 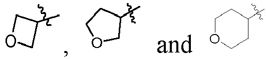 , above groups are optionally further substituted with one or more substituents selected from the group consisting of deuterium, fluoro, chloro, bromo, cyano, nitro, azido, methyl, ethyl, isopropyl, difluoromethyl, trifluoromethyl, dichloromethyl, trichloromethyl, dibromomethyl, tribromomethyl, dideuteriomethyl, trideuteriomethyl, hydroxymethyl, cyclopropyl, cyclobutyl, cyclopentyl, hydroxycyclopropyl, 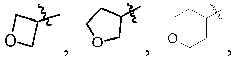 hydroxy, methoxy, ethoxy, carboxy, methoxyacyl and ethoxyacyl.

3. The compound of formula (I), the stereoisomer, prodrug or pharmaceutically acceptable salt thereof according to claim 1, wherein the compound of formula (I) is a compound having formula (IIa), formula (IIb), formula (IIc), formula (IId) or formula (IIe): 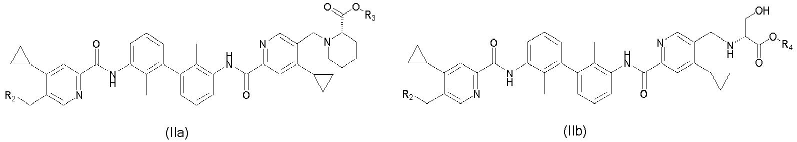 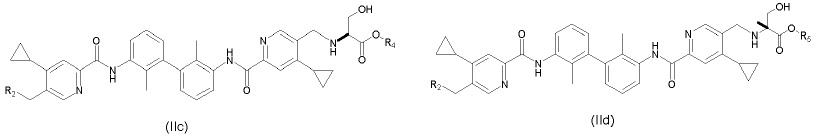 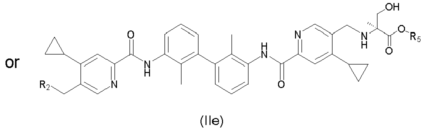 wherein R₂ is -NHR₆ or 4-6 membered nitrogen-containing heterocyclyl, wherein a nitrogen atom is linked to methylene,
provided that R₂ in the compound of formula (II a) is not 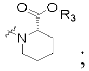
R₂ in the compound of formula (IIb) is not 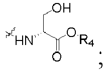
R₂ in the compound of formula (II c) is not 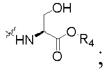
the 4-6 membered nitrogen-containing heterocyclyl is selected from the following structures: 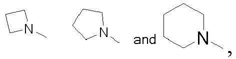 above groups are optionally further substituted with one or more substituents selected from the group consisting of deuterium, fluoro, chloro, bromo, cyano, methyl, ethyl, isopropyl, cyclopropyl, hydroxycyclopropyl, hydroxymethyl, hydroxy, methoxy, ethoxy, carboxy, methoxyacyl and ethoxyacyl;
R₃, R₄ and R₅ are each independently selected from the group consisting of hydrogen, deuterium, methyl and ethyl;
R₆ is selected from the group consisting of methyl, ethyl, isopropyl, cyclopropyl, cyclobutyl, cyclopentyl, cyclohexyl. 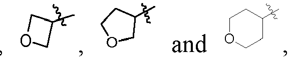 above groups are optionally further substituted with one or more substituents selected from the group consisting of deuterium, fluoro, chloro, bromo, cyano, methyl, ethyl, isopropyl, cyclopropyl, hydroxycyclopropyl, hydroxymethyl, hydroxy, methoxy, ethoxy, carboxy, methoxyacyl and ethoxyacyl.

4. The compound of the formula (I), the stereoisomer, prodrug or pharmaceutically acceptable salt thereof according to claim 1, wherein the compound is selected from the following compounds:

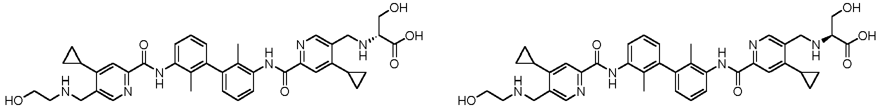

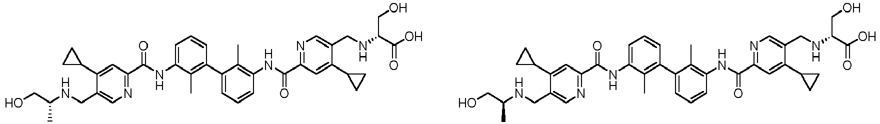

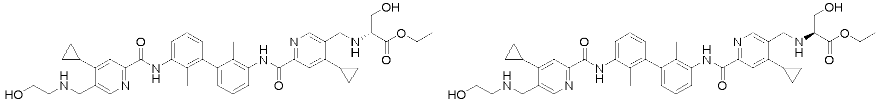

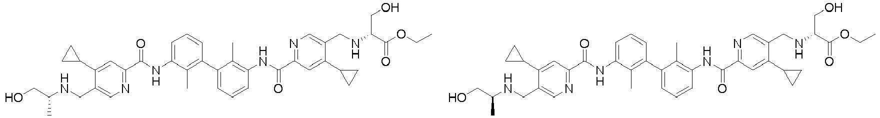

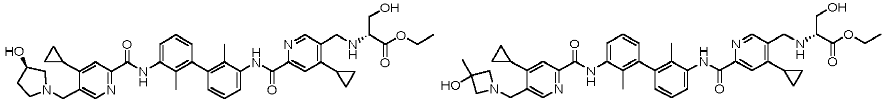

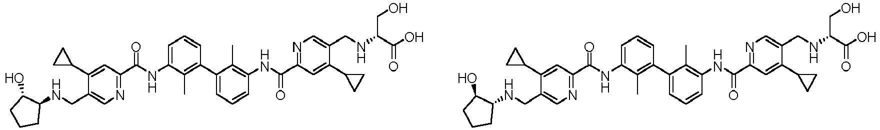

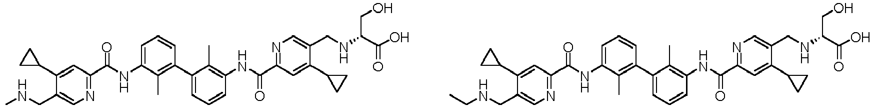

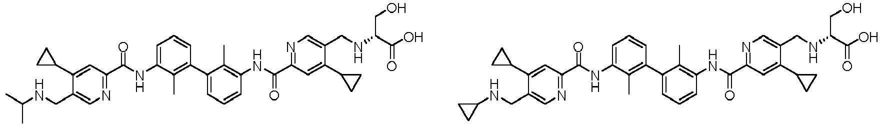

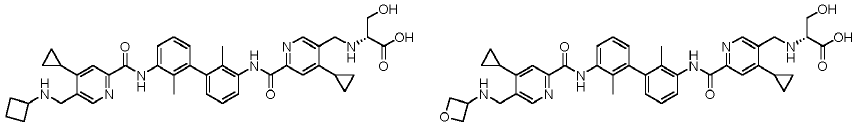

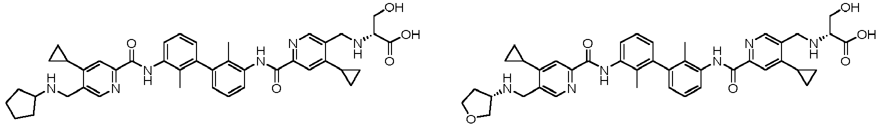

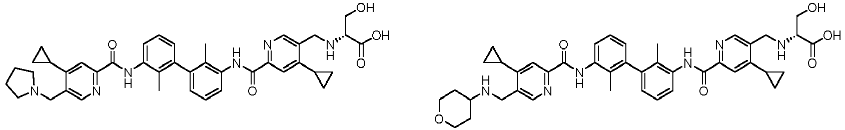

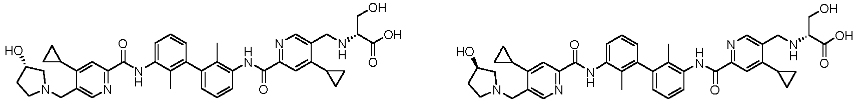

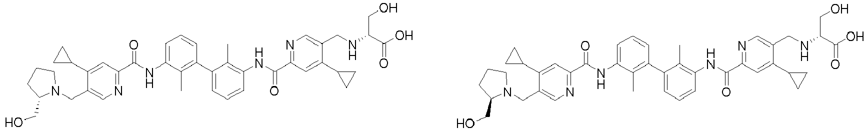

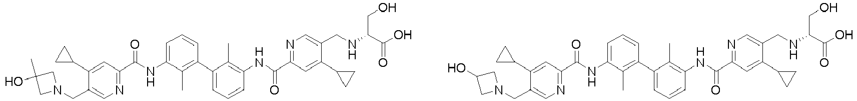

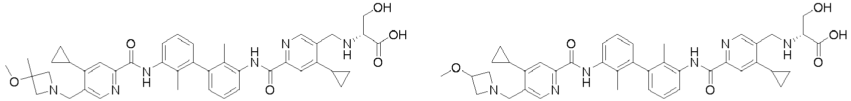

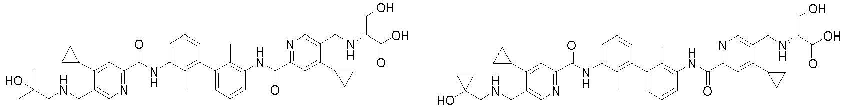

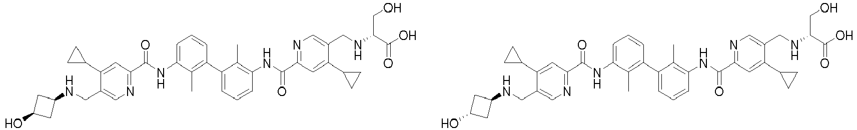

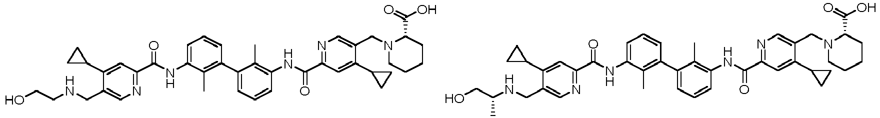

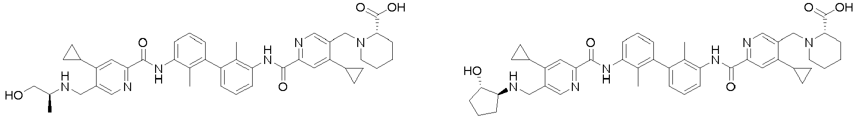

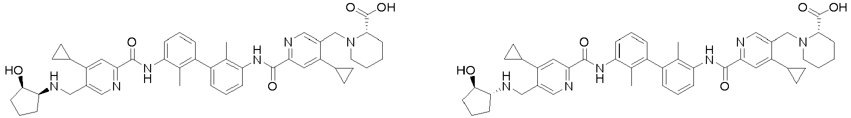

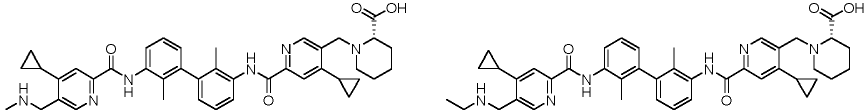

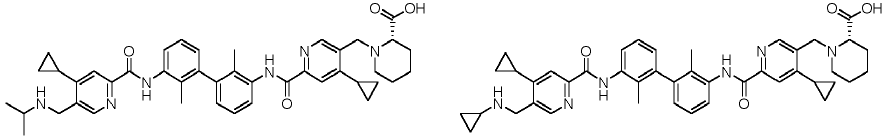

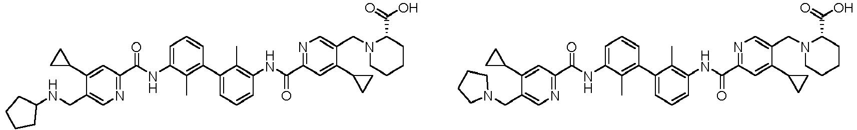

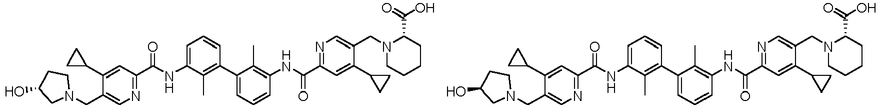

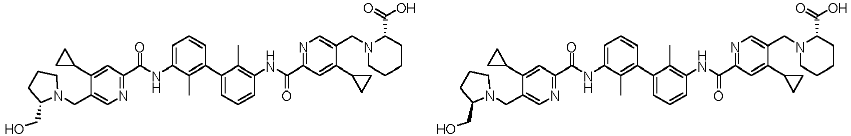

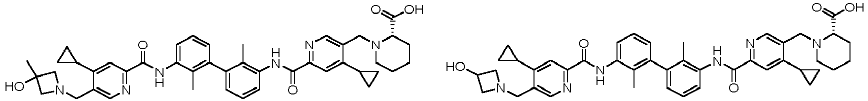

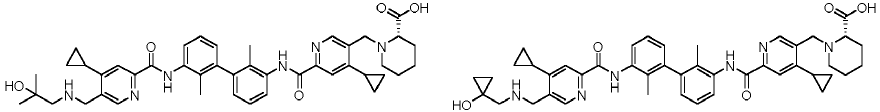

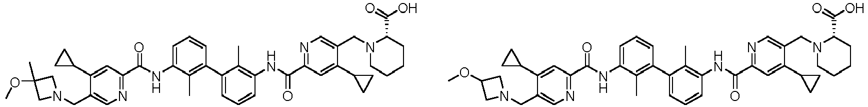

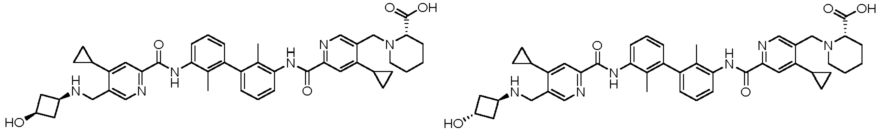

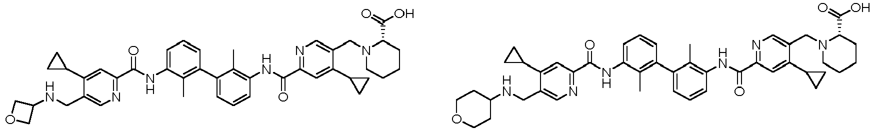

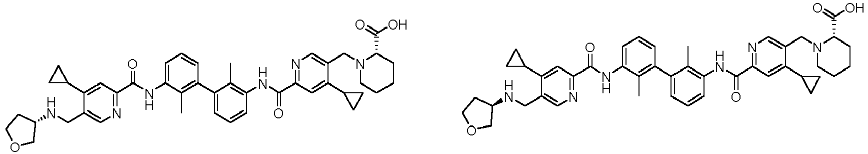

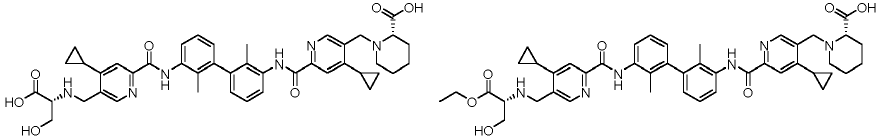

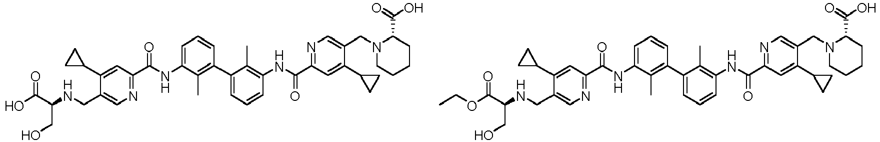

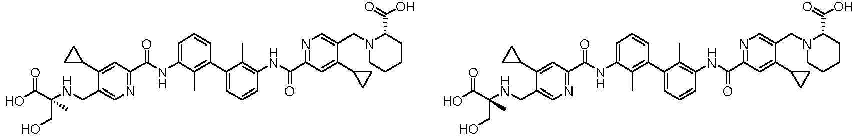

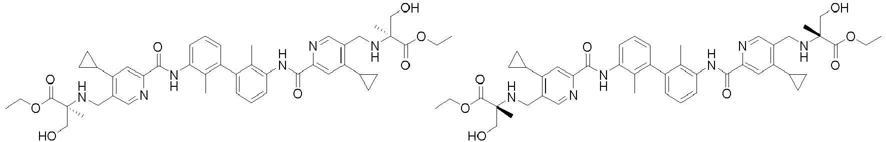

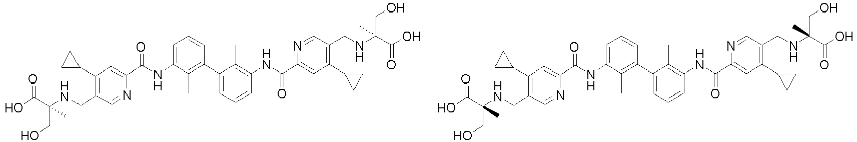

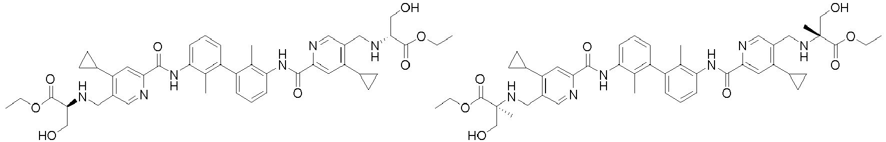

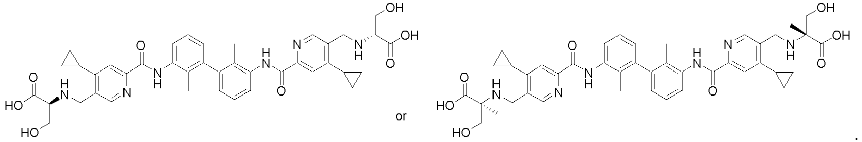

5. A method for preparing the compound of formula (I), the stereoisomer, prodrug or pharmaceutically acceptable salt thereof according to any one of claims 1 to 4, comprising the following steps: 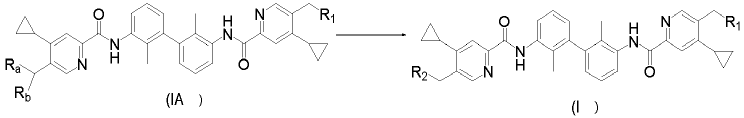 or, 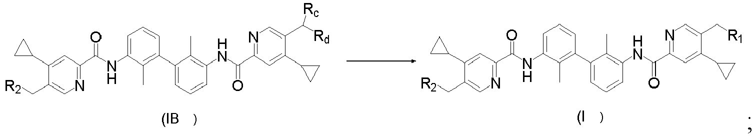 wherein, Rₐ, R_{b}, R_{c} and R_{d} are each independently C₁₋₈ alkoxy, or Rₐ together with R_{b}, R_{c} together with R_{d} is =O, and R₁ and R₂ are defined as in claim 1.

6. A pharmaceutical composition, comprising the compound of formula (I), the stereoisomer, prodrug or pharmaceutically acceptable salt thereof according to any one of claims 1 to 4, and a pharmaceutically acceptable carrier.

7. Use of the compound of formula (I), the stereoisomer, prodrug or pharmaceutically acceptable salt thereof according to any one of claims 1 to 4 in the preparation of a medicament for preventing and/or treating a PD-1/PD-L1 signal pathway-mediated disease.

8. The use according to claim 7, wherein the PD-1/PD-L1 signal pathway-mediated disease is cancer or tumor, immune-related disease and disorder, communicable disease, infectious disease or metabolic disease.

9. The use according to claim 8, wherein the infectious disease is bacterial infectious disease, viral infectious disease and fungal infectious disease; the cancer or tumor is lymphoma (including but not limited to lymphocytic lymphoma, primary central nervous system lymphoma, T cell lymphoma, diffuse large B cell lymphoma, follicle center lymphoma, Hodgkin lymphoma, non-Hodgkin lymphoma or primary mediastinal large B cell lymphoma), sarcoma (including but not limited to Kaposi's sarcoma, fibrosarcoma, liposarcoma, chondrosarcoma, osteosarcoma, leiomyosarcoma, rhabdomyosarcoma, soft tissue sarcoma, angiosarcoma or lymphangiosarcoma), melanoma, glioblastoma, synovioma, meningioma, biliary tract tumor, thymic tumor, neuroma, seminoma, nephroblastoma, pleomorphic adenoma, hepatocellular papilloma, renal tubule adenoma, cystadenoma, papilloma, adenoma, leiomyoma, rhabdomyoma, hemangioma, lymphangioma, osteoma, chondroma, lipoma, fibroma, central nervous system tumor, rhachiophyma, brain stem glioma, pituitary adenoma, multiple myeloma, ovarian tumor, myelodysplastic syndrome or mesothelioma, prostate cancer, recurrent prostate cancer or prostate cancer having developed resistance to existing medicaments, thyroid cancer, parathyroid cancer, anal cancer, testicular cancer, urethral carcinoma, penile cancer, bladder cancer, ureteral cancer, uterine cancer, ovarian cancer, fallopian tube cancer, endometrial cancer, cervical cancer, vaginal cancer, vulvar cancer, adrenal cancer, Merkel cell carcinoma, embryonal carcinoma, chronic or acute leukemia (including but not limited to acute myeloid leukemia, chronic myeloid leukemia, acute lymphoblastic leukemia, chronic granulocytic leukemia and chronic lymphoblastic leukemia), bronchial carcinoma, esophageal cancer, nasopharyngeal carcinoma, hepatocellular carcinoma, renal cell carcinoma, small cell lung cancer, basal cell carcinoma, lung cancer, breast cancer, adenocarcinoma, papillary carcinoma, cystadenocarcinoma, squamous non-small cell lung cancer, non-squamous non-small cell lung cancer, rectal cancer, colon cancer, colorectal cancer, gastric cancer, pancreatic cancer, head and neck squamous cell carcinoma, head and neck cancer, gastrointestinal cancer, bone cancer, skin cancer, small intestine cancer, endocrine system cancer, renal pelvic carcinoma, epidermoid carcinoma, abdominal wall carcinoma, renal cell carcinoma, transitional cell carcinoma, choriocarcinoma, or metastatic tumor, especially metastatic tumor expressing PD-L1;
the immune-related disease and disorder is rheumatic arthritis, renal failure, lupus erythematosus, asthma, psoriasis, ulcerative colitis, pancreatitis, allergy, fibrosis, anemia, fibromyalgia, Alzheimer's disease, congestive heart failure, stroke, aortic valve stenosis, arteriosclerosis, osteoporosis, Parkinson's disease, infection, Crohn's disease, ulcerative colitis, allergic contact dermatitis and eczema, systemic sclerosis or multiple sclerosis;
the communicable disease or infectious disease is sepsis, liver infection, HIV, hepatitis A, hepatitis B, hepatitis C, hepatitis D, herpes virus, papillomavirus or influenza;
the metabolic disease is diabetes, diabetic ketoacidosis, hyperglycemic hyperosmolar syndrome, hypoglycemia, gout, malnutrition, vitamin A deficiency, scurvy, vitamin D deficiency or osteoporosis.

10. The compound of formula (I), the stereoisomer, prodrug or pharmaceutically acceptable salt thereof according to any one of claims 1 to 4 for use as a medicament for preventing and/or treating PD-1/PD-L1 signal pathway-mediated cancer or tumor, immune-related disease and disorder, communicable disease, infectious disease or metabolic disease.
